# EUROPEAN PATENT APPLICATION

(11) **EP 4 086 276 A1**
(43) Date of publication of application: **09.11.2022**
(21) Application number: 21305569.2
(22) Date of filing: 03.05.2021
(51) Int. Cl.: C07K 14/47, A61K 48/00, A61P 21/00

(54) **COMPOSITION FOR TREATING DYSFERLINOPATHY**

(71) Applicant: Université d'Aix-Marseille, 13007 Marseille (FR); INSERM - Institut National de la Santé et de la Recherche Médicale, 75013 Paris (FR)
(72) Inventor: BARTOLI, Marc, 13190 Allauch (FR); COURRIER, Sébastien, 13109 Simiane-Collongue (FR); BALLOUHEY, Océane, 13005 Marseille (FR)
(74) Representative: IPAZ

(57) **Abstract**

The present invention pertains to gene therapy for treating dysferlinopathy. More particularly, the invention relates to a polynucleotide sequence comprising several domains of dysferlin, or functional variants thereof, to a viral vector for gene therapy comprising at least a polynucleotide sequence of the invention exon 40a of the coding sequence of dysferlin.

## Description

### FIELD OF THE INVENTION

The present invention pertains to gene therapy for treating dysferlinopathy. More particularly, the invention relates to a polynucleotide sequence comprising several domains of dysferlin, or functional variants thereof, it also relates to a viral vector for gene therapy comprising at least a polynucleotide sequence of the invention exon 40a of the coding sequence of dysferlin.

### TECHNICAL BACKGROUND

Dysferlinopathies are autosomal recessive disorders caused by mutations in the dysferlin (DYSF) gene, encoding the dysferlin protein. Dysferlin is a modular type II transmembrane protein containing seven calcium sensor C2 domains that play a key role in muscle membrane repair. DYSF mutations lead to a wide range of muscular phenotypes, in particular Miyoshi myopathy (MM), that affects distal muscles and limb girdle muscular dystrophy type 2B (LGMD2B or LGMDR2) characterized by proximal weakness. Symptoms generally appear at the end of childhood and, although disease progression is typically slow, walking impairment eventually result.

Various therapeutic approaches for treating myopathies or dysferlinopathies have been proposed so far, although no curative treatment for dysferlinopathies exist.

WO 2020/123645 discloses a combination therapy for treating muscular dystrophy, wherein adenovirus gene therapy vectors comprise a polynucleotide encoding a first polypeptide, or a first RNA, and a second polypeptide, or a second RNA. This combination therapy involves both gene restoration and reduction of symptoms associated with a number of secondary cascades such as fibrosis.

WO 2018/170408 discloses an adeno-associated virus vector delivery of muscle specific micro-dystrophin, to treat muscular dystrophy. The gene therapy vectors are used to raise muscular strength and/or prevent fibrosis in patients affected by muscular dystrophy.

WO 2011/054659 discloses an exon-skipping therapy for dysferlinopathies, by a process comprising a step of preventing the splicing of one or more exons coding for amino acid sequences responsible of dysferlin dysfunction. Said exon is in particular exon 32. Complementary antisense oligonucleotides used for correcting said splicing are also disclosed.

Barthelemy et al ("Translational research and therapeutic perspectives in dysferlinopathies", Mol. Med. 17(9-10) 875-882, sept-oct, 2011) is a review describing therapeutic perspectives in dysferlinopathies. Barthelemy discloses a "mini-dysferlin" comprising the last two C2 domains of dysferlin and the transmembrane domain.

There is therefore a strong need for active therapy of dysferlinopathies.

The inventors have now shown that a protein comprising an amino acid sequence encoded by exon 40a of dysferlin protein is a key factor in the membrane repair process, and participates in other muscle cell functions, like muscle cell membrane protection and protein vesicle trafficking.

The present invention therefore relates to a nucleic acid sequence encoding for essential domains of dysferlin, including a nucleic acid sequence, such as exon 40a (GenBank EF015906), encoding for a cleavage site by calpain. A nucleic acid sequence of the invention is therefore useful to restore muscle cells repairing function in patients with dysferlinopathy.

Krahn *et al* ("*Exclusion of mutations in the dysferlin alternative exons 1 of DYSF-vl, 5a and 40a in a cohort of 26 patients",* Gen. Test. Mol. Biomarkers, 2010, Feb. 14(1); 153-4, DOI 10.1089/gtmb.2009.0131) disclose a screening of mutations causing primary dysferlinopathies in three known alternative exons, DYSF-v1, 5a and 40a. The authors conclude that no disease-causing mutation was identified in said alternative exons, demonstrating a low frequency of disease-causing mutations in these exons.

Redpath et al ("Calpain cleavage within dysferlin exon 40a releases a synaptotagmin-like module for membrane repair", MBoC, vol. 25, Oct. 1, 2014) disclose that injury-activated cleavage of dysferlin is mediated by the ubiquitous calpains via a cleavage motif encoded by alternately spliced exon 40a.

### SUMMARY OF THE INVENTION

The inventors have now developed a nucleic acid sequence comprising, or consisting of, from its 5' to 3' extremity:
a) a nucleic acid sequence encoding for at least 100 amino acids of the dysferlin N-terminal amino acids N°1 to 301 (SEQ ID N°1) or for an amino acid sequence exhibiting at least 80% identity with SEQ ID N°1,
b) a nucleic acid sequence encoding for a polypeptide comprising a cleavage site by calpain, and
c) a nucleic acid sequence encoding for the dysferlin C2F, C2G and TM domains (SEQ ID N°2), for an amino acid sequence exhibiting at least 80% identity with said SEQ ID N°2 or for an amino acid sequence comprising at least 100 amino acids of SEQ ID N°2.

A nucleic acid sequence according to the invention codes for a polypeptide which is useful to prevent or abolish symptoms of dysferlinopathy, when injected to a patient in need thereof. A nucleic acid sequence according to the invention codes for a polypeptide useful to restore cell membrane repair, cell membrane protection and/or vesicle trafficking. In particular, a nucleic acid sequence according to the invention codes for a polypeptide able to restore cell membrane repair.

In a particular embodiment of the invention, a nucleic acid sequence of the invention encodes for a protein designated as "Midi-dysferlin" (SEQ ID N°4), a functional fragment thereof or a functional variant thereof.

The inventors have also developed a recombinant expression vector comprising at least a nucleic acid sequence according to the invention. A recombinant expression vector according to the invention can be used as a gene therapy vector. It is submitted that a recombinant expression vector according to the invention at least allows the restoration of the phenotype of a dysferlin deficient cell in an animal model, and may restore the "calpain cleavage activation" of dysferlin in a patient in need thereof.

The invention is based on the surprising discovery that the polypeptide encoded by exon 40a is strongly involved in the dysferlin repairing process of muscle cells membranes through the presence of a cleavage site by calpain, and on the observation that some mutations in said exon 40a sequence do not abolish the functional property of said peptide to form a cleavage site by calpain.

The inventors consequently defined a domain comprising a cleavage site by calpain, wherein said domain is either a polypeptide encoded by exon 40a, a functional fragment thereof or a functional variant thereof, or a polypeptide domain known in the art as susceptible to be cleaved by calpain.

The present invention therefore provides a gene replacement therapy aimed to at least partially restore dysferlin functions. A vector of the present invention therefore helps to reduce or prevent at least one symptom of a dysferlinopathy.

### DETAILED DESCRIPTION OF THE INVENTION

In a first aspect, the present invention relates to an isolated nucleic acid sequence comprising, or consisting of, from its 5' to 3' extremity:
a) a nucleic acid sequence encoding the dysferlin amino acids N°1 to 301 (SEQ ID N°1), for an amino acid sequence exhibiting at least 80% identity with SEQ ID N°1, or for an amino acid sequence comprising at least 100 amino acids of SEQ ID N°1.
b) a nucleic acid sequence encoding for a polypeptide comprising a cleavage site by calpain, and
c) a nucleic acid sequence encoding for the dysferlin C2F, C2G and TM domains (SEQ ID N°2), for an amino acid sequence exhibiting at least 80% identity with said SEQ ID N°2 or for an amino acid sequence comprising at least 100 amino acids of SEQ ID N°2.

The gene encoding human dysferlin (DYSF, Online Mendelian Inheritance in Man (OMIM) gene number 603009, 2p13, GenBank NM_003494.2) encodes several transcripts, the most common transcript is composed of 55 exons and alternative splicing events generate transcript diversity, such as exon 1, exon 5a and exon 40a (GenBank EF015906)

From its N-terminal to its C-terminal extremity, the human dysferlin protein comprises:
- dysferlin amino acids N°1 to 301 comprising C2A and C2B domains, wherein said C2A and C2B domains bind to phospholipids, with C2A domain binding to phospholipids in a calcium dependent manner;
- dysferlin amino acids N°381 to 1992 comprising C2C, C2D, C2E, C2F and C2G domains,
- dysferlin amino acids N°2045 to 2067 comprising a transmembrane domain (TM).

By "a nucleic acid sequence encoding for at least 100 amino acids" of a given nucleotide or amino-acid sequence, it is intended a nucleic acid sequence encoding for at least 100, 150, 200, 250, 280, 290 or 300 of said amino acid sequence.

By "exhibiting at least 80% identity" is meant that said sequences exhibit at least 80% identity after optimal overall alignment, that is to say by global alignment between two sequences giving the highest percentage identity. between them. The optimal global alignment of two sequences can in particular be carried out according to the Needleman-Wunsch algorithm, well known to those skilled in the art (Needleman & Wunsch, "A general method applicable to the search for similarities in the amino acid sequences of two proteins", J. Mol. Biol., 48 (3): 443-53).

Proteins encoding by a nucleic acid according to the invention comprise, or consist of an amino acid sequence having at least 80%, advantageously at least 81%, 82%, 83%, 84%, 85%, 86%, 87%, 88%, 89%, 90%, 91%, 92%, 93%, 94%, 95%, 96%, 97%, 98% or 99% identity with the amino acid sequence SEQ ID N °1 after global alignment optimal. Advantageously, the proteins according to the invention comprise, or consist of an amino acid sequence exhibiting at least 99.1%, 99.2%, 99.3%, 99.4%, 99.5%, 99.6%, 99.7%, 99.8% or 99.9% identity with said SEQ ID N°1

In a particular embodiment of this aspect, a nucleic acid sequence according to the invention encodes for a polypeptide exhibiting, at least partially, functional properties of said polypeptide having an amino acid sequence SEQ ID N°1.

By "exhibiting, at least partially, functional properties of said polypeptide" it is intended a polypeptide exhibiting at least 10%, 15%, 20%, 30%, 40% or 50% of the functional properties of said polypeptide, expressed as a measure or an estimation of the percentage of the functional property of a polypeptide having an amino acid sequence SEQ ID N°1.

By "functional properties" of a polypeptide exhibiting at least 80% identity with SEQ ID N°1, it is intended:
- an ability to bind to membrane surfaces phospholipids, in particular to phosphoinositide and to phosphatidylserine, possibly in a calcium dependent manner, and
- an ability to bind to proteins involved in membrane repair, in particular to tubulin and to MG53.

Calpains (code CAPN for Calcium Activated Neutral Proteases) are non-lysosomal cysteine proteases exhibiting a cytosolic papain-like activity controlled by calcium. Calpains are expressed ubiquitously in mammals. Today, 14 different calpains are described, including calpain 1 and calpain 2. The calpain proteolytic system includes the calpain proteases and the small regulatory subunit CAPNS1, also known as CAPN4.

By "a nucleic acid sequence encoding for a polypeptide comprising a cleavage site by calpain" it is intended a nucleic acid sequence encoding for a polypeptide which is cleaved by the calpain proteolytic enzyme, which is naturally present in the cells, under appropriate reaction conditions. Among calpains susceptible to cleave said polypeptide, calpain 1 and/or calpain 2 are preferred.

Appropriate reaction conditions are defined as follows:
- an incubation of myoblasts (at 70% of confluency) with ionomycine (30 µM) at room temperature for 3 minutes and then
- an incubation in PBS 1X supplemented with calcium 1 mM at 37°C during 3 minutes to allow muscle repair mechanism.

Proteins of the treated myoblasts are then extracted and analysed by SDS-PAGE.

It has been shown that the cleavage of dysferlin by calpain leads to the presence of mini-dysferlin. Therefore, the presence of a cleavage site by calpain can be demonstrated by the presence of mini-dysferlin after said cleavage reaction. More generally, the presence of a cleavage site by calpain can be demonstrated by the presence of a protein of the appropriate size after said cleavage reaction.

It is estimated that an ability to being cleaved by calpain, even at a low level, is acceptable for a polypeptide encoded by a nucleic acid sequence of the invention. In particular, an ability to being cleaved by calpain of at least 5%, 10%, 15%, 20%, 30%, 40% or 50% of the property of the wild-type polypeptide encoded by exon 40a is acceptable.

Among nucleic acid sequences encoding for a polypeptide comprising a cleavage site by calpain, a particular embodiment of the invention relates to nucleic acid sequences encoding for the cleavage site by calpain of spectrin (SEQ ID N°6).

Among nucleic acid sequences encoding for a polypeptide comprising a cleavage site by calpain, a particular embodiment of the invention relates to nucleic acid sequences encoding for:
- a polypeptide exhibiting the amino acid sequence (SEQ ID N°4) or an amino acid sequence exhibiting at least 80% identity with SEQ ID N°4,
- a polypeptide exhibiting the amino acid sequence (SEQ ID N°5) or an amino acid sequence exhibiting at least 80% identity with SEQ ID N°5,
- a polypeptide exhibiting the amino acid sequence (SEQ ID N°6) or an amino acid sequence exhibiting at least 80% identity with SEQ ID N°6.

More particularly, nucleic acid sequences encoding for a polypeptide exhibiting the amino acid sequence (SEQ ID N°4) or for an amino acid sequence exhibiting at least 80% identity with SEQ ID N°4 are preferred.

The inventors have shown that some polypeptides exhibiting mutated sequences of SEQ ID N°4 are still able to be cleaved by calpain, these polypeptides exhibit an amino acid sequence chosen among: SEQ ID N°8, SEQ ID N°9, SEQ ID N°10, SEQ ID N°11, SEQ ID N°12, SEQ ID N°13, SEQ ID N°14, SEQ ID N°15, SEQ ID N°16, SEQ ID N°17, SEQ ID N°18 and SEQ ID N°19.

Consequently, in a particular embodiment of the invention, a nucleic acid sequence encoding for a polypeptide comprising a cleavage site by calpain encodes for a polypeptide exhibiting an amino acid sequence chosen among: SEQ ID N°4, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16, SEQ ID N°17, SEQ ID N°18 and SEQ ID N°19.

In a preferred embodiment, a nucleic acid sequence encoding for a polypeptide comprising a cleavage site by calpain encodes for a polypeptide exhibiting an amino acid sequence SEQ ID N°4.

By "a nucleic acid sequence encoding for the dysferlin C2F, C2G and TM domains (SEQ ID N°2) it is intended a nucleic acid sequence encoding for the amino acids N°1563 to 2080 of dysferlin, which comprises domains C2F, C2G and TM of said dysferlin protein.

According to this aspect, a nucleic acid sequence encoding for at least 100 amino acids of the dysferlin N-terminal amino acids of SEQ ID N°2 or for an amino acid sequence exhibiting at least 80% identity with SEQ ID N°1, exhibits, at least partially, functional properties of SEQ ID N°2.

In a particular embodiment, the present invention relates to an isolated nucleic acid sequence comprising, or consisting of, from its 5' to 3' extremity:
a) a nucleic acid sequence encoding for at least 100 amino acids of the dysferlin N-terminal amino acids N°1 to 301 (SEQ ID N°1) or for an amino acid sequence exhibiting at least 80% identity with SEQ ID N°1, wherein said nucleic acid sequence encodes for the amino acid sequence of C2A domain (SEQ ID N°3), or for an amino acid sequence exhibiting at least 80% identity with SEQ ID N°3.
b) a nucleic acid sequence encoding for a polypeptide comprising a cleavage site by calpain, and
c) a nucleic acid sequence encoding for the dysferlin C2F, C2G and TM domains (SEQ ID N°2), for an amino acid sequence exhibiting at least 80% identity with said SEQ ID N°2 or for an amino acid sequence comprising at least 100 amino acids of SEQ ID N°2.

In another particular embodiment, the present invention relates to an isolated nucleic acid sequence comprising, or consisting of, from its 5' to 3' extremity:
i. a nucleic acid sequence encoding for at least 100 amino acids of the dysferlin N-terminal amino acids N°1 to 301 (SEQ ID N°1) or for an amino acid sequence exhibiting at least 80% identity with SEQ ID N°1,
ii. a nucleic acid sequence encoding for a polypeptide comprising a cleavage site by calpain, wherein said nucleic acid sequence encodes for a polypeptide comprising a cleavage site by calpain, said polypeptide comprising or consisting of the amino acid sequence SEQ ID N°4, a functional fragment thereof comprising at least a sequence SEQ ID N°5, or an amino acid sequence exhibiting at least 80% identity with SEQ ID N°6,
iii. a nucleic acid sequence encoding for the dysferlin C2F, C2G and TM domains (SEQ ID N°2), for an amino acid sequence exhibiting at least 80% identity with said SEQ ID N°2 or for an amino acid sequence comprising at least 100 amino acids of SEQ ID N°2.

In another particular embodiment, the present invention relates to an isolated nucleic acid sequence comprising, or consisting of, from its 5' to 3' extremity:
i. a nucleic acid sequence encoding for at least 100 amino acids of the dysferlin N-terminal amino acids N°1 to 301 (SEQ ID N°1) or for an amino acid sequence exhibiting at least 80% identity with SEQ ID N°1,
ii. a nucleic acid sequence encoding for a polypeptide comprising a cleavage site by calpain, wherein said nucleic acid sequence encoding for a cleavage site by calpain is nucleic acid sequence SEQ ID N°20, corresponding to dysferlin exon 40a, a nucleic acid sequence exhibiting at least 80% identity with SEQ ID N°A or a nucleic acid sequence comprising at least 30 nucleotides of SEQ ID N°20,
iii. a nucleic acid sequence encoding for the dysferlin C2F, C2G and TM domains (SEQ ID N°2), for an amino acid sequence exhibiting at least 80% identity with said SEQ ID N°2 or for an amino acid sequence comprising at least 100 amino acids of SEQ ID N°2.

In another particular embodiment, the present invention relates to an isolated nucleic acid sequence comprising, or consisting of, from its 5' to 3' extremity:
i. a nucleic acid sequence encoding for the dysferlin C2A domain (SEQ ID N°3),
ii. a nucleic acid sequence corresponding to exon 40a (SEQ ID N°20), and
iii. a nucleic acid sequence encoding for the dysferlin C2F, C2G and TM domains (SEQ ID N°2).

In another particular embodiment, the present invention relates to an isolated nucleic acid sequence comprising, or consisting of the nucleic acid sequence SEQ ID N°21, or a nucleic acid sequence exhibiting at least 80 %, at least 85%, 90%, 95%, 98% identity with SEQ ID N°21.

In a second aspect, the present invention relates to a recombinant vector comprising at least an isolated nucleic acid sequence according to the invention. More particularly, the present invention relates to a recombinant viral vector comprising at least an isolated nucleic acid sequence according to the invention

The present invention relates to a recombinant viral vector comprising at least:
I. a nucleotide according to the invention, and
II. a control element, in particular a muscle-specific control element, operably linked to and driving the expression of said polynucleotide, wherein said control element is preferably a promoter.

In vivo gene therapy is a direct method of inserting the genetic material into the targeted tissue, and transduction takes place within the transfected cells. A gene therapy vector according to the invention is desirably delivered locally, or systematically.

Among possible gene therapy vectors appropriate for the invention, viral vectors are particularly indicated. More particularly, the present invention relates to a recombinant viral vector comprising at least an isolated nucleic acid sequence according to the invention, for gene therapy.

Adeno-associated virus (AAV) is a replication-deficient parvovirus, the single stranded DNA genome of which is about 4,7 kb in length, including 145 nucleotides inverted terminal repeat (ITR).

AAV possesses many features that make it attractive for delivering foreign DNA to cells, in particular for gene therapy. Multiple studies have demonstrated long-term recombinant AAV-mediated protein expression in muscle and that the muscle is capable of stable expression of secreted protein therapeutics.

As known by a person skilled in the art of designing and manipulating vectors for gene therapy, and particularly AAV, grows only in cells in which certain functions are provided by a co-infecting helper virus.

Recombinant AAV genomes of the invention comprises nucleic acid molecules of the invention and one or more AAV ITRs flanking a nucleic acid molecule.

Reported clinical doses for AAV-based viral vectors range from 10¹¹ to 10¹⁴ vector genomes per patient, depending on therapeutic area.

In a particular embodiment, a recombinant vector of the invention is a viral vector. In a more particular embodiment, a recombinant vector of the invention is a recombinant AAV (rAAV) vector or a recombinant lentiviral vector.

In a more particular embodiment, the vector is of the serotype AAV1, AAV2, AAV3, AAV4, AAV5, AAV6, AAV7, AAV8, AAV9, AAV10, AAV11, AAV12 or AAV13 serotype, or a derivative of a known serotype. In certain embodiment, the vector exhibits a desired tissue specific or tropism or other desirable properties for a pharmaceutical composition or gene therapy for various indications.

In another particular embodiment, the muscle-specific control element is human skeletal actin gene element, a human myosin kinase promoter or a synthetic muscle specific promoter C5.12.

In certain embodiments, a vector of the invention comprises a poly-A adenylation sequence for inserting a polyA sequence into a transcribe mRNA.

In a third aspect, the present invention relates to a composition comprising a recombinant vector according to the invention and a therapeutically acceptable carrier, diluent or excipient.

In a forth aspect, the present invention relates to a recombinant vector comprising at least an isolated nucleic acid sequence according to the invention or a composition comprising a recombinant vector according to the invention, for its use as a medicament.

More particularly, the present invention relates to a recombinant vector comprising at least an isolated nucleic acid sequence according to the invention or a composition comprising a recombinant vector according to the invention, for its use as a medicament for the treatment of a dysferlinopathy.

Vectors suitable for the invention can be produced by using any the methods known in the art. Viral particles can be produced by using any the methods known in the art such as using stable mammalian cell lines.

Another aspect of the invention provides a method of producing a viral vector of the invention, comprising culturing a cell that has been transfected with a viral vector of the invention and recovering the viral particles from the supernatant of the transfected cells.

Another aspect of the invention provides viral particles comprising any of the viral vectors of the invention.

Another aspect of the invention provides a composition comprising a vector according to the invention.

According to an embodiment, a composition according to the invention is a pharmaceutical composition further comprising a therapeutically compatible carrier, diluent or excipient.

For administration, effective amounts and therapeutically effective amounts, also referred as doses, may be initially estimated based on results from in vitro assays and/or animal model studies. A vector according to the present invention, when injected to a mouse model, such as for example a deficient-dysferlin mouse model (Dysf Y1159X/Y1159X), leads to the production of a recombinant protein; analysis of expressed protein and the quantification of dystrophic features lead to the characterization of the polypeptide encoded by said vector according to the invention.

The actual dose of vector according to the invention will vary according to the particular vector used, the mode of administration, the treatment goal, the individual and the cell types being targeted, and may be determined by methods standard in the art. The actual dose of vector according to the invention may also be determined by a physician taking into account physical and physiological factors, severity of condition and/or route of administration. Exemplary doses may range from about 1x10¹⁰ to about 1x10¹⁵ vector genomes per kilogram of body weight.

According to an embodiment, a pharmaceutical composition according to the invention is in a dosage form comprising at least 5x10¹¹ vector genomes, more preferably from about 5x10¹¹ to about 10¹⁵ vector genomes.

The present invention also provides a vector according to the invention, or a composition according to the invention, for its use as a medicament.

The present invention also provides a vector according to the invention, or a composition according to the invention, for its use as a medicament for treating dysferlinopathy.

Even more particularly, the present invention relates to a recombinant vector comprising at least an isolated nucleic acid sequence according to the invention or a composition comprising a recombinant vector according to the invention, for its use as a medicament for the treatment of a dysferlinopathy chosen among Miyoshi myopathy (MM), limb girdle muscular dystrophy (LGMD) and limb girdle muscular dystrophy type 2B (LGMD2B or LGMDR2).

According to an embodiment, the present invention provides a vector according to the invention, or a composition according to the invention, for its use as a medicament for treating Miyoshi myopathy (MM) or limb girdle muscular dystrophy type 2B (LGMD2B or LGMDR2).

In a particular embodiment, a vector according to the invention or a composition is in a form suitable for intramuscular injection, intravenous injection, parental delivery or systemic administration.

The present invention also provides a method for the treatment of dysferlinopathy in a subject in need thereof, the method comprising administering to the subject a therapeutically effective amount of a vector according to the invention or a composition according to the invention.

The following examples illustrates, without limiting it, the key role played by a polynucleotide encoding for exon 40a in cellular processes of dysferlinopathy, supporting the medical solution provided by a gene therapy vector according to the invention and its use for treating dysferlinopathy.

Any one embodiment described herein can be combined with any one or more other embodiments of the invention, unless such combination is expressly disclaimed or improper.

Characteristics, advantages and uses of the subject-matter of the present invention are more detailed hereunder, in an illustrated and non-limiting way. When present, the disclosure of the ranges expressed as "from ... to ..." mean that the limits are included in said ranges.

### BRIEF DESCRIPTION OF THE FIGURES:

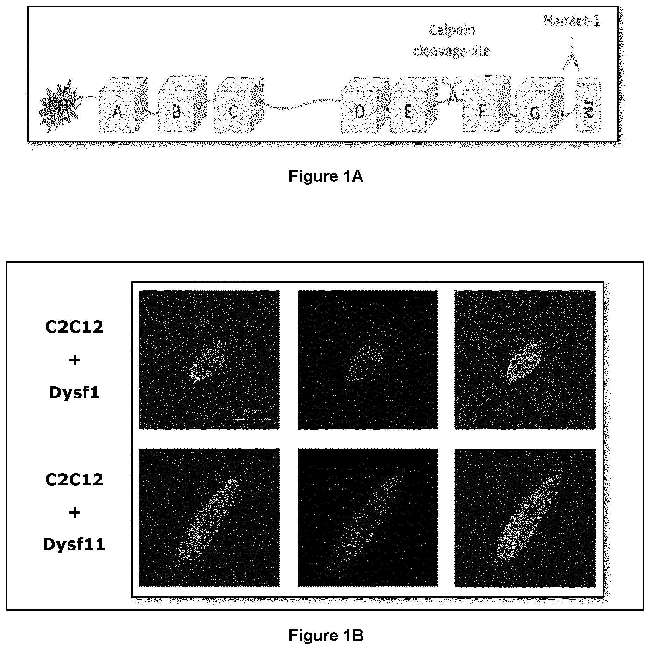

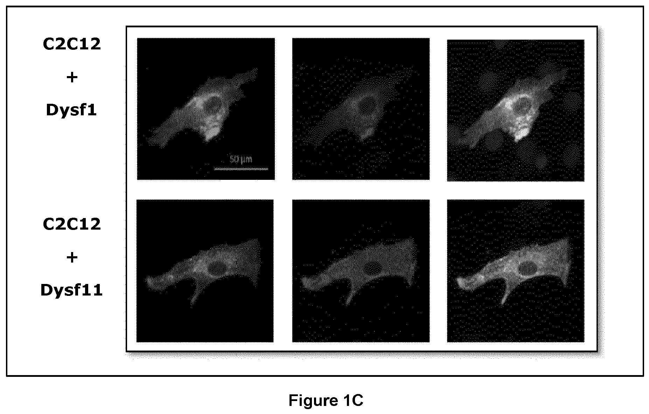

Figures 1A, 1B and 1C show the location difference between N-ter and C-ter part of dysferlin11 transcript. Fig. 1A represents the dysferlin, the GFP and Hamlet-1 binding site and the cleavage site by calpain. Fig. 1B and Fig. 1C represent respectively dysferlin labeling performed on transfected and uninjured (Fig. 1B) or transfected and injured (Fig. 1C). In both Fig. 1B and Fig. 1C, upper images represent murine myoblasts C2C12 transfected with dysferlin1 (which does not comprise Exon 40a) and lower images represent C2C12 cells transfected with dysferlin11 (which does comprise Exon 40a). In both Fig. 1B and Fig. 1C, left images represent GFP labeling, middle images represent Hamlet-1 labeling and right images represent merge labeling. DAPI was used as a nucleus marker (blue). All images were captured by an apotome microscope. Scale bar, 100µM.

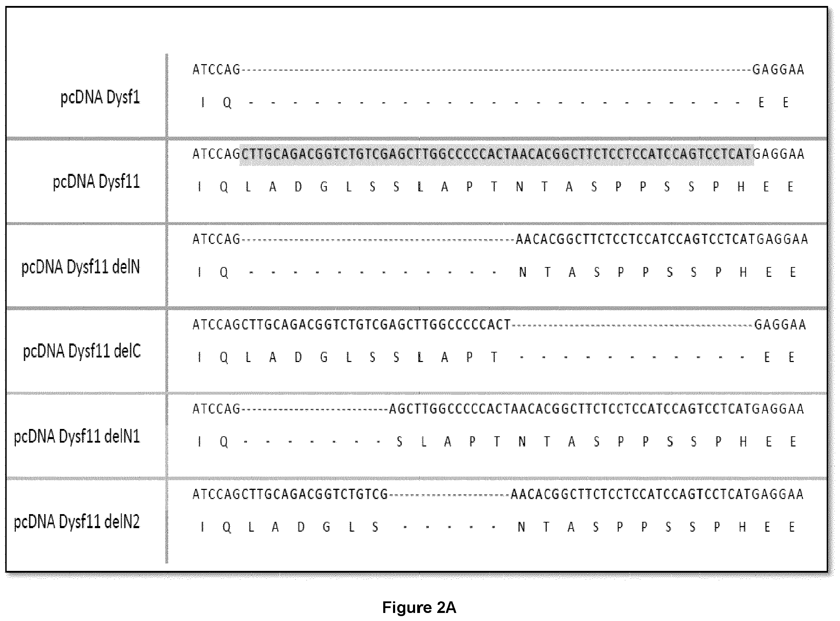

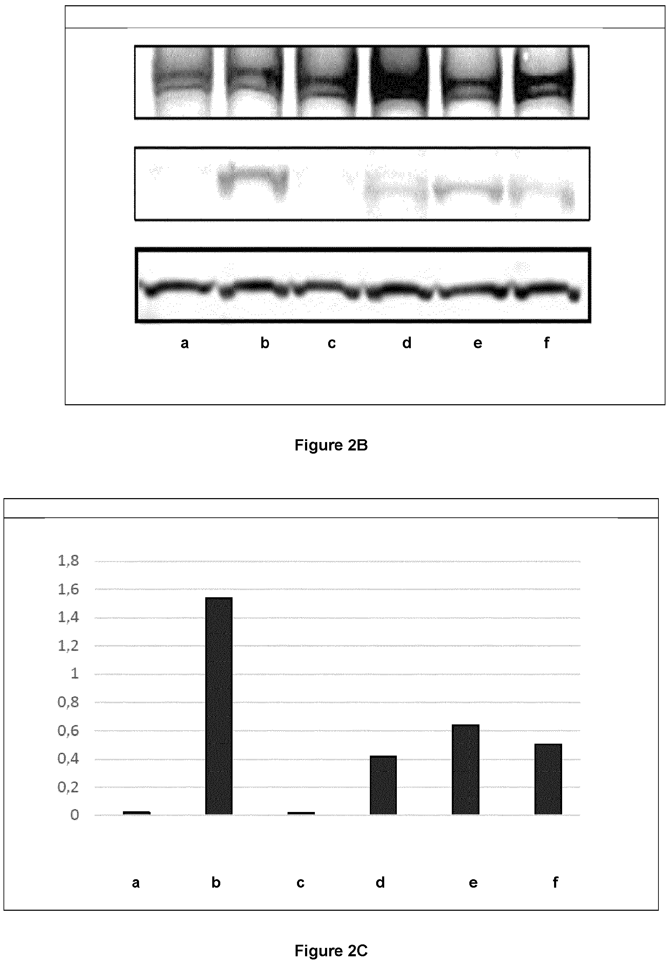

Figures 2A, 2B and 2C illustrate the importance of the N-terminal part of *DYSF* exon 40a for calpain cleavage. Figure 2A represents the different dysferlin11 constructs created to identify cleavage site by calpain in *DYSF* exon 40a. Constructs were built by PCR fusion. Figure 2B represents a western blot performed with proteins from HEK cells transfected with dysferlin constructs and injured with ionomycin and cells scrapers 24h post-transfection. Hamlet was used to detect dysferlin and dysferlin cleaved (respectively upper and middle part of Fig. 2B). Actin was used for normalization (lower Fig. 2B). Figure 2C represents the normalized ratio of cleaved dysferlin for each construction, results show that only dysferlin transcript 11 was able to be cleaved by calpain. For Fig. 2B and Fig 2C, "a" is for Dysf1, "b" for Dysf11, "c" for Dysf11delN, "d" for Dysf11delC, "e" for Dysf11delN1, "f" for Dysf11delN2.

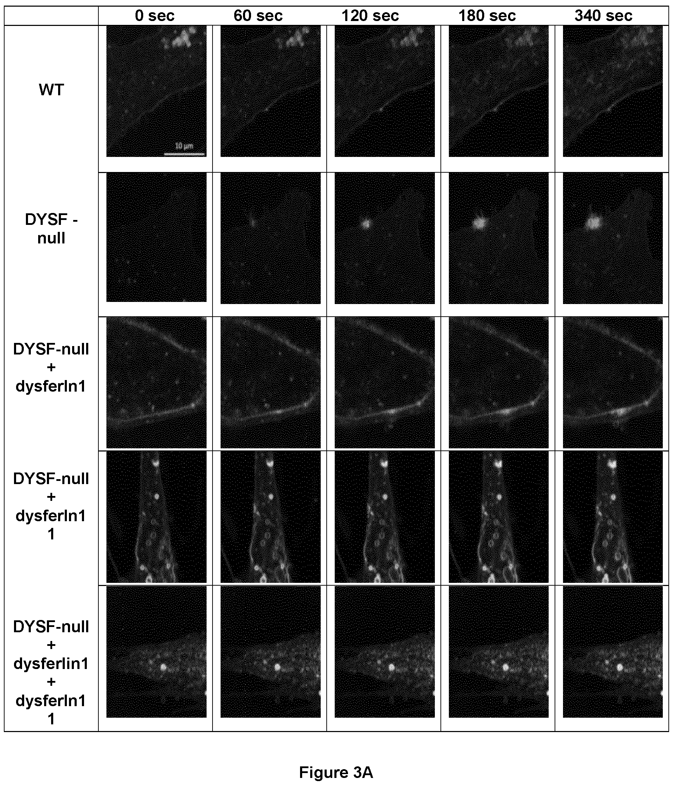

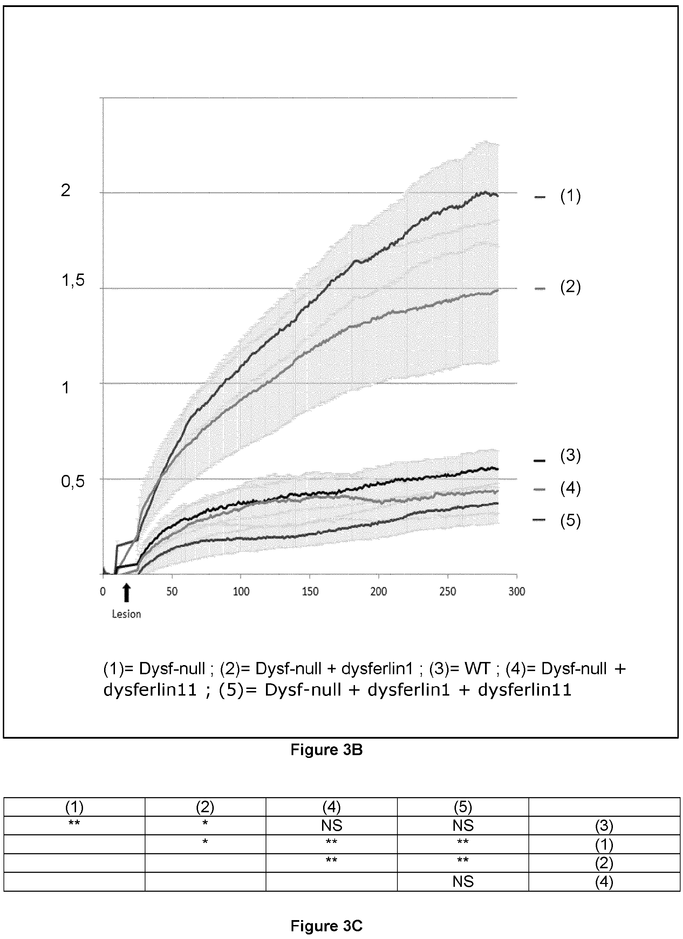

Figures 3A, 3B and 3C illustrate the importance of dysferlin11 transcript for muscle cell membrane repair. Fig. 3A: Membrane repair assay performed on wild-type myoblasts (WT, C25), dysferlin-null myoblasts (DYSF-null, AB320) and transfected dysferlin-null myoblasts (DYSF-null + mCherry-dysferlin1, DYSF-null + mCherry-dysferlin11, DYSF-null + mCherry-dysferlin1 + mCherry-dysferlin11) in the presence of Ca²⁺ and membrane-impermeable dye FM 1-43. Fluorescence images were taken every second for 5 minutes. The damage was induced at t = 10 sec. Scale bar, 10µM. White arrows indicate injury site. Fig. 3B: Summary data for membrane repair assay. The black arrow indicates injury time point. Data are mean ± SEM (Standard error of the mean) for n = 20 cells. Statistical analysis was done with Mann-Whitney test (NS: non-significant, *: p-value < 0.05, **: p-value < 0.01). Fig. 3C presents the results of statistical analysis.

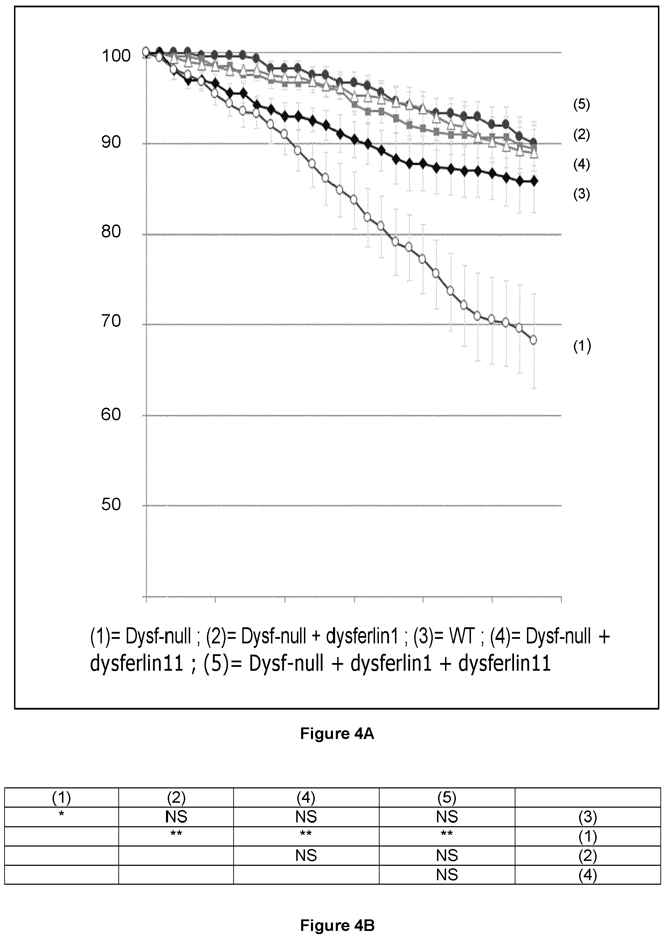

Figures 4A and 4B shows that dysferlin11 transcript participates in the protection of membrane myoblasts from mechanical stress induced by osmotic shock and represents the percentage of cell survival as a function of time (Fig. 4A) and the results of statistical analysis (Fig. 4B). Osmotic shock assay performed on wild-type myoblasts (WT, C25), dysferlin-null myoblasts (DYSF-null, AB320) and transfected dysferlin-null myoblasts (DYSF-null + GFP-dysferlin1, DYSF-null + GFP-dysferlin11, DYSF-null + GFP-dysferlin1 + GFP- dysferlin11) in hypotonic solution (ultrapure distilled water). Fluorescence images were taken every minute for 30 minutes. Medium change was done at t = 1 minute. Data are mean ± SEM (Standard error of the mean) for n = 10. For each experiment, at least 20 cells were observed. Statistical analysis was done with Mann-Whitney test (NS: non-significant, *: p-value < 0.05, **: p-value < 0.01).

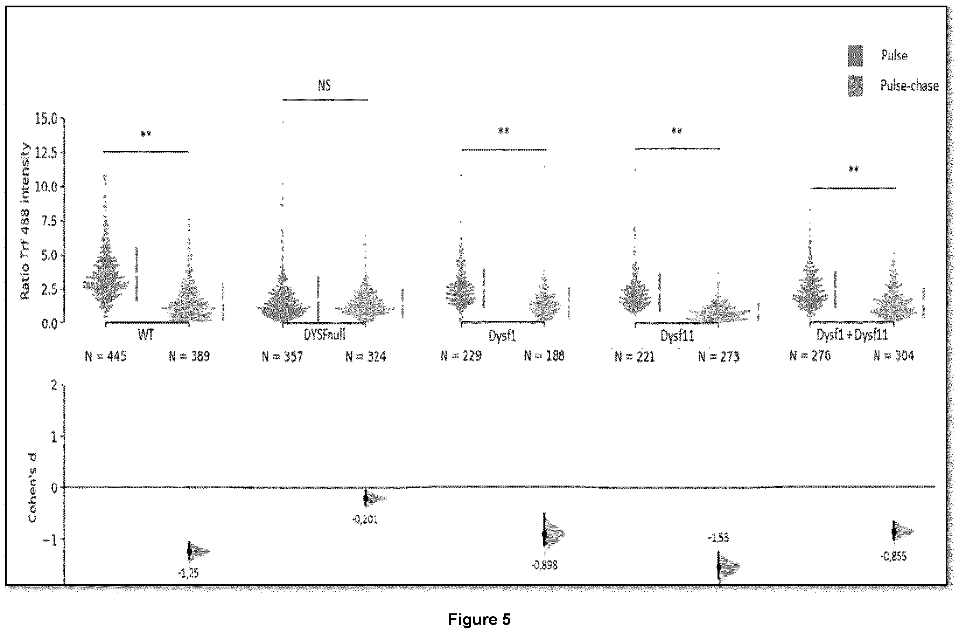

Figure 5 illustrates the intensity of transferrin in each cell (wherein a dot represents a cell) in the different reaction conditions (WT, DYSF-null or transfected DYSF-null cells) and shows that dysferlin11 transcript participates in protein vesicle trafficking in muscle cell. Transferrin assay performed on wild-type myoblasts (WT, C25), dysferlin-null myoblasts (DYSF-null, AB320) and transfected dysferlin-null myoblasts (DYSF-null + mCherry- dysferlin1, DYSF-null + mCherry-dysferlin11, DYSF-null + mCherry-dysferlin1 + mCherry-dysferlin11). Myoblasts were incubated for 30 min with transferrin coupled with Alexa Fluor 488 (pulse) and for 30 min with transferrin-free medium (pulse-chase). For each condition, transferrin intensity was measured in more than 180 cells observed in three experiments. Statistical analysis was done with Mann-Whitney test (NS: non-significant, *: p-value < 0.05, **: p-value < 0.01) and Cohen test.

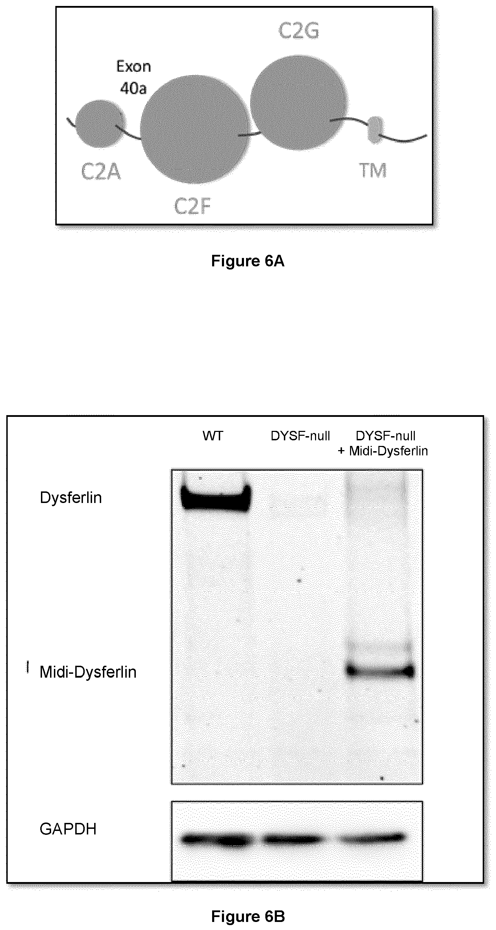

Figures 6A and 6B represent respectively the schematic construction of "midi-dysferlin", said polypeptide comprising, from its Nt to Ct extremities, a C2A domain, a polynucleotide encoded by Exon 40a, C2F and C2G domains and transmembrane domain ^{™} (Fig. 6A) and a western blot (Fig. 6B) performed with proteins from wild-type myoblasts (WT, C25), dysferlin- null myoblasts (DYSF-null, AB320) and transfected dysferlin- null myoblasts (DYSF-null + Midi-dysferlin), Hamlet was used to detect dysferlin and midi-dysferlin, GAPDH was used for normalization.

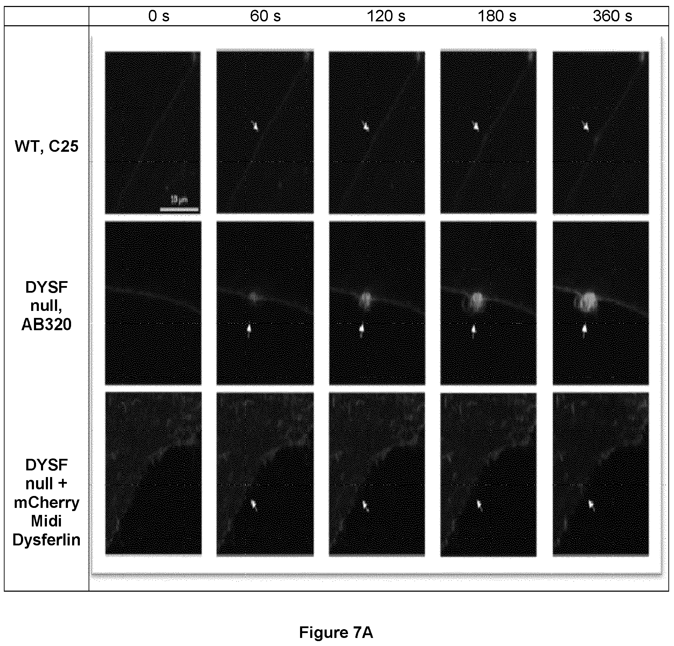

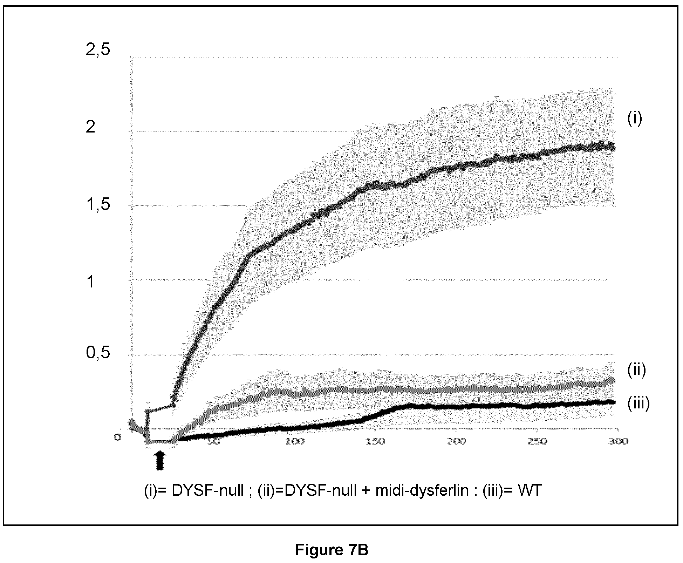

Figures 7A and 7B represent respectively a membrane repair assay (Fig. 7A) performed on wild-type myoblasts (WT, C25, upper images), dysferlin-null myoblasts (DYSF-null, AB320, middle images) and transfected dysferlin-null myoblasts (DYSF-null + mCherry-midi-dysferlin, lower images) in the presence of Ca²⁺ and membrane-impermeable dye FM 1-43. Fluorescence images were taken every second for 5 minutes, wherein images represent from left to right, images taken at 0, 60, 120, 180 and 360 seconds. The damage was induced at t = 10 sec. Scale bar is 10µM. White arrows indicate injury site. Fig. 7B represents summary data for membrane repair assay, wherein the ratio DF / F0 is represented as a function of time (in seconds), the upper black curve represents DYSF-null, the intermediate grey curve represents DYSF-null + midi-dysferlin, the lower black curve represents WT. Black arrow « lesion » indicate injury time point. Data are mean ± SEM (Standard error of the mean) for n = 20 cells. Statistical analysis was done with Mann-Whitney test (NS: non-significant, *: p-value < 0.05, **: p-value < 0.01).

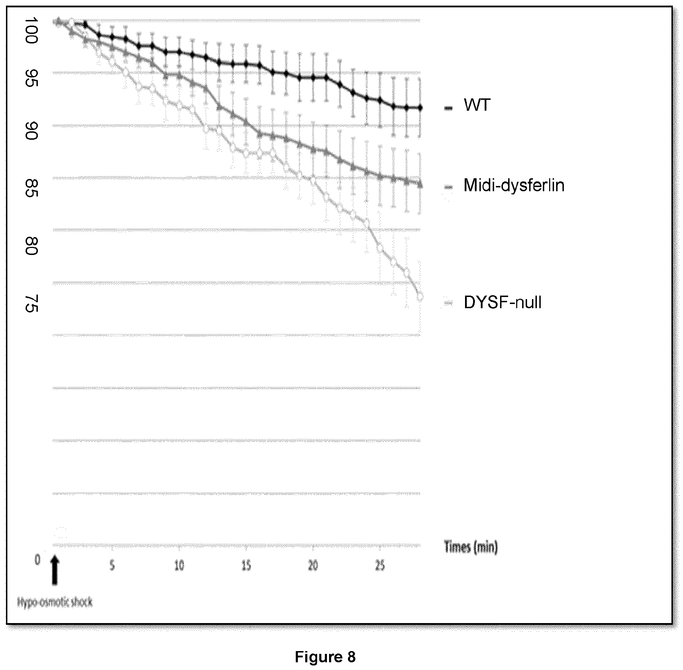

Figure 8 represents the cell survival ratio as a function of time in an osmotic shock assay performed on wild-type myoblasts (WT, C25), dysferlin-null myoblasts (DYSF-null, AB320) and transfected dysferlin-null myoblasts (DYSF-null + GFP-midi-dysferlin) in hypotonic solution (ultrapure distilled water). Fluorescence images were taken every minute for 30 minutes. Medium change was done at t = 1 minute. Data are mean ± SEM (Standard error of the mean) for n = 10. For each experiment, at least 20 cells were observed. Statistical analysis was done with Mann-Whitney test (NS: non-significant, *: p-value < 0.05, **: p-value < 0.01).

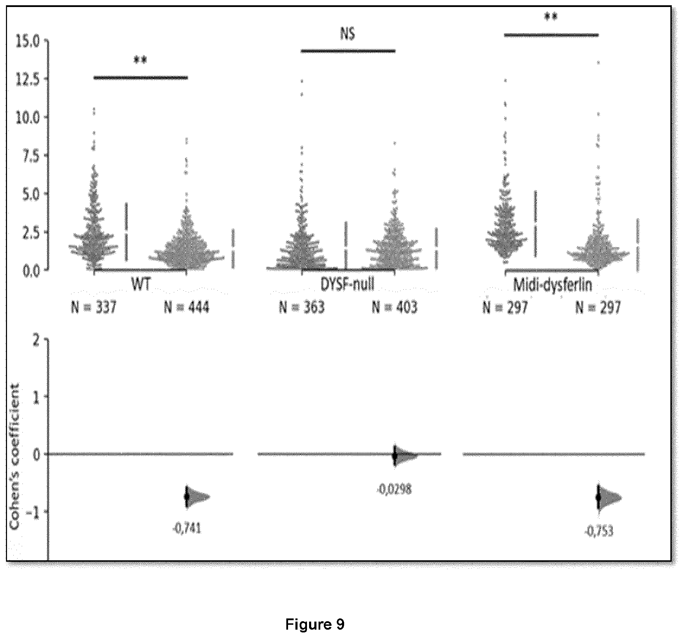

Figure 9 represents transferrin intensity in a transferrin assay performed on wild-type myoblasts (WT, C25, left), dysferlin-null myoblasts (DYSF-null, AB320, middle) and transfected dysferlin-null myoblasts (DYSF-null + mCherry-midi-dysferlin, right). Myoblasts were incubated for 30 min with transferrin coupled with Alexa Fluor 488 (« pulse" dots, left) and for 30 min with transferrin-free medium ("pulse-chase" right). For each condition, transferrin intensity was measured in more than 290 cells observed in three experiments. Statistical analysis was done with Mann-Whitney test (NS: non-significant, *: p-value < 0.05, **: p-value < 0.01). The lower part of Fig. 9 represents Cohen's coefficient for each condition with respectively: for WT: WT pulse-chase minus WT pulse, for dysferlin-null myoblasts: DYSF-null pulse-chase minus DYSF-null pulse, and for transfected dysferlin-null myoblasts: midi-dysferlin pulse-chase minus midi-dysferlin pulse.

### EXAMPLES

### EXAMPLE 1: Characterization of the role of alternative exon 40a of dysferlin

### 1. Material and methods

### Cell cultures

WT (C25) and DYSF-null (AB320) human myoblasts cells lines were kindly given by Vincent Mouly from the Myology Research Center (Paris, France). Cells were grown in a humidified environment at 37°C and 5% CO2 in Dulbecco's modified Eagle's medium, supplemented with 15% medium 199, 15% fetal bovine serum, 25µg/mL fetuin, 5ng/mL hEGF, 0.5ng/mL bFGF, 5µg/mL insulin and 0.2µg/mL dexamethasone.

C2C12 murine myoblasts and HEK cells lines were bought at ATCC. Cells were grown in a humidified environment at 37°C and 5% CO₂ in Dulbecco's modified Eagle's medium (ThermoFisher), supplemented with 20% fetal bovine serum and 100µg/mL antibiotic antimycotic (GE Healthcare, ref P11-002).

### Plasmid construction

GFP-dysferlin1 plasmid was a generous gift from Dr. Kate Bushby, it contains the transcript of the main isoform of dysferlin (exon 1 to 55). mCherry-dysferlin1 plasmid was generated by GFP switching to mCherry (Shaner et al, Nature Biotech., 21 nov. 2004) using the EcoRI and KpnI restriction enzymatic sites.

Coding sequence of GFP-dysferlin11 was generated by Cliniscience by inserting the alternative exon 40a between exon 40 and exon 41 into the GFP-dysferlin1 plasmid to reproduce the transcript 11 of the dysferlin. mCherry-dysferlin11 plasmid was generated by GFP switching to mCherry using the EcoRI and KpnI restriction enzymatic sites. Dysferlin11 constructs to identify the cleavage site by calpain in DYSF exon 40a were created by PCR fusion. Table 1 summarizes the primers used to create dysferlin11 constructs by PCR fusion.

**Table 1.**

| | | |
|---|---|---|
| pcDNA Dysf11 delN | Forward | GCCCCTCATCCCCATCCAGAACACGGCTTCTCCTCCATCCAGTCCTC |
| | Reverse | GATGGAGGAGAAGCCGTGTTCTGGATGGGGATGAGGGGCTCCTTGTCATC |
| pcDNA Dysf11 delC | Forward | GTCGAGCTTGGCCCCCACTGAGGAAGAGTTCATCGATTGGTGGAGC |
| | Reverse | CCAATCGATGAACTCTTCCTCAGTGGGGGCCAAGCTCGACAGACCGTC |
| pcDNA Dysf11 delN1 | Forward | CCTCATCCCCATCCAGAGCTTGGCCCCCACTAACACGGCTTCTCC |
| | Reverse | GTTAGTGGGGGCCAAGCTCTGGATGGGGATGAGGGGCTCCTTGTCATC |
| pcDNA Dysf11 delN2 | Forward | GCTTGCAGACGGTCTGTCGAACACGGCTTCTCCTCCATCCAGTCC |
| | Reverse | GGAGGAGAAGCCGTGTTCGACAGACCGTCTGCAAGCTGGATGGGG |

### Cell scraping injury, SDS-PAGE and Western Blot

HEK cells were plated at 70% confluence in 6-well plate (VWR) and transfected using Lipofectamine 2000 (Invitrogen) per manufacturer's directions. After 24h, cells were injured with 30µM ionomycin and cell scrappers, then they were pelleted at 300g for 5 min and the cell pellet was solubilized in RIPA buffer (Life technologies) and protease inhibitor cocktail (Life technologies). Samples were separated by SDS-PAGE on 3-8% NuPAGE Tris-Acetate gels (Life technologies) using Chameleon Duo as a size marker and transferred onto nitrocellulose membranes (at 100V for 3h at 4°C). Membranes were blocked using fluorescent WB blocking buffer (tebu-bio) in TBS 1X for 1 hour at room temperature. Primary antibodies (Hamlet, 1:150, abcam 75571) were then diluted in blocking buffer and incubated overnight at 4°C. After washing in TBS-T, membranes were then incubated with secondary antibodies (IRDye 800CW Donkey anti-mouse, Li-Cor, ref 926-32212), which were diluted 1:10000 in blocking buffer for 45 min at room temperature. The membranes were washed in TBS-T and developed using NIR Fluorescence LI-COR. Actin (1:5000, Merck MA1501r) was detected using a dilution of 1:10000 of secondary antibodies (IRDye 680RD Donkey anti-mouse, Li-Cor, ref 925-68072).

### Immunofluorescence

C2C12 myoblasts were grown on Lab-TEK II^{™} (Fisher Scientific) and transfected using Lipofectamine 2000 (Invitrogen) per manufacturer's directions. After 24h, cells were injured with glass beads, fixed with 4% paraformaldehyde for 10 minutes and then washed in PBS. Cells were then incubated for 10 minutes with a permeabilization solution (200 µL of PBS 1X +0.5% triton X-100+protease inhibitors cocktail) (Roche). From there, cells were exposed to a blocking buffer (PBS+ 1% BSA + protease inhibitors cocktail) for 30 minutes. The primary antibody (Hamlet, 1:40, abcam 75571) was applied in blocking buffer for 3 hours at room temperature, followed by a wash in PBS and 1 hour of contact with the secondary antibody (Dylight 550 donkey anti mouse IgG, 1:100, abcam 96876) in blocking buffer. After washing in PBS, Lab-TEK II^{™} were mounted with Vectashield-Dapi 25ng/mL and kept at 4°C until pictures were taken. Observation was performed using a Zeiss Axio Imager Z2 microscope (40X objective), and images were processed with ZEN software and/or ImageJ software.

### Membrane repair assay

WT (C25) and DYSF-null (AB320) human myoblasts were plated at 50% confluence on 6-well plate (VWR) and transfected using Lipofectamine 2000 (Invitrogen) per manufacturer's directions. After 24h, cells were visualized in the presence of Ca²⁺ (1mM) and membrane-impermeable dye FM 1-43 (2.5µM, Molecular Probes) with a confocal microscope (LSM 800, 63X objective, ZEISS). Membrane damage was applied to myoblast using a UV laser to irradiate a 0.33 µm² area at maximum power for 15 seconds at t = 10 sec. Images were captured every second for 5 min, and the mean fluorescence intensity of FM1-43 was measured on a 13.5 µm² area around the damage with the Zeiss LSM 800 imaging software.

### Osmotic shock assay

WT (C25) and DYSF-null (AB320) human myoblasts were plated at 70% confluence on 12-well plate (VWR) and transfected using Lipofectamine 2000 (Invitrogen) per manufacturer's directions. After 24h, myoblasts were washed with distilled water and hypo- osmotic shock was performed by incubating cells with distilled water. Cell fluorescence was followed with Fast calcium imaging observer (Axio observer. Z1/7, 10X objective, ZEISS). Images were captured every minute for 30 minutes.

### Transferrin uptakes and recycling assays

WT (C25) and DYSF-null (AB320) human myoblasts were plated at 70% confluence on Lab-TEK II^{™} (Fisher Scientific) and transfected using Lipofectamine 2000 (Invitrogen) per manufacturer's directions. After 24h, myoblasts were incubated in Dulbecco's modified Eagle's medium, supplemented with 1% L-glutamine and 0.5% bovine serum albumin at 37°C and 5% CO₂ for 30 minutes. Then, cells were incubated in the same medium with 25µg/mL transferrin from human serum (coupled with Alexa Fluor 488, Thermo Scientific) at 37°C and 5% CO₂ for 30 minutes. For transferrin recycling assays, cells were washed in cold PBS and incubated in Dulbecco's modified Eagle's medium, supplemented with 15% medium 199, 15% fetal bovine serum, 25µg/mL fetuin, 5ng/mL hEGF, 0.5ng/mL bFGF, 5µg/mL insulin and 0.2µg/mL dexamethasone. Then, myoblasts were washed in PBS and incubated in stripping buffer (NaCl, acid acetic) during a few seconds. After that, cells were washed in PBS, fixed with 4% paraformaldehyde for 10 minutes and washed again in PBS. Lab-TEK II^{™} were mounted with Vectashield-Dapi 25ng/mL and kept at 4°C until pictures were taken. Observation was performed using a Zeiss Axio Imager Z2 microscope (20X objective), and images were processed with ZEN software and/or ImageJ software.

### Statistical analysis

Individual means were compared using the non-parametric Mann-Whitney test. The power of the tests was strictly superior at 92%. Differences were considered to be statistically significant if p<0.05 (*) or very significant if p<0.01 (**).

### 2. Results

Dysferlinopathy encompasses muscular dystrophies caused by mutations in the DYSF gene. Dysferlin gene encodes a sarcolemmal protein required for repairing muscle cell damage. It consists of calcium-dependent lipid binding domains and a transmembrane domain. Dysferlin-deficient muscle fibers have a defect in membrane repair.

### 2.1. Cleavage site by calpain in DYSF exon 40a

Several publications demonstrated that membrane injury causes calcium influx at injury sites which induces local activation of calpains. This activation led to dysferlin cleavage by calpains. In 2014, it has been shown that the cleavage site by calpain resides in the *DYSF* exon 40a. To identify the exact location of cleavage site by calpain in dysferlin exon 40a, four plasmids containing the dysferlin transcript 11 with deletions of fragments in exon 40a were constructed: Dysf11delN, Dysf11delC, Dysf11 delN1, Dysf11delN2 (Figure 3A). Injury assays by ionomycin and cell scrappers were performed in HEK cells and followed dysferlin cleavage by western blot analysis with Hamlet-1 antibody (Figure 3B). Protein extracted from injured cells transfected with dysferlin transcript 11 revealed the presence of the mini-dysferlin band as the expected size whereas in injured HEK transfected with dysferlin transcript 1 no signal for the mini-dysferlin was detected.

When exon 40a is deleted in two parts (one on the N-terminal DelN side and one on the C- terminal DelC side), deletion of the N-terminal part had a hardly effect on the intensity of the band corresponding to the mini-dysferlin. Thus, the N-terminal part would be more important than the C-terminal part for the recognition and cleavage of this region by calpains. Mutations and small deletions were performed in *DYSF* exon 40a. Surprisingly, none of the mutations tested abolishes the cleavage of this domain by calpains, as represented in Table 2.

**Table 2**

| ID | Exon 40a modification | Calpain cleavage |
|---|---|---|
| S1485A | CTTGCAGACGGTCTGTCGAGCTTGGCCCCCACTAACACGGCTGCTCCTGCATCCAGTCCTCAT | Yes |
| P14S7A | | |
| S1485A | CTTGCAGACGGTCTGTCGAGCTTGGCCCCCACTAACACGGCTGCTCCTGCATCCGGTCCTCAT | Yes |
| P1487A | | |
| S14S9G | | |
| S1475Y | CTTGCAGACGGTCTGTACAGCTTGGCCCCCACTAACACGGCTTCTCCTCCATCCAGTCCTCAT | Yes |
| S1476A | CTTGCAGACGGTCTGGCGSGCTTGGCCCCCACTAACACGGCTTCTCCTCCATCCAGTCCTCAT | Yes |
| 51477G | | |
| T1481A | CTTGCAGACGGTCTGTCGAGCTTGGCCCCCGCTAACACGGCTTCTCCTCCATCCAGTCCTCAT | Yes |
| T1483K | CTTGCAGACQGTCTGTCGAGCTTGGCCCCCACTAACAAGGCTTCTCCTCCATCCAGTCCTCAT | Yes |
| 51488Y | CTTGCAGACGGTCTGTCGAGCTTGGCCCCCACTAACACGGCTTCTCCTCCAACCAGTCCTCAT | Yes |
| Del PTNT | CTTGCAGACGGTCTGTCGAGCTTGGCC-----------GCTTCTCCTCCATCCAGTCCTCAT | Yes |
| Del P1480 | CTTGCAGACGGTCTGTCGAGCTTGGCC-----ACTAACACGGCTTCTCCTCCATCCAGTCCTCAT | Yes |
| P14805 | CTTGCAGACGGTCTGTCGAGCTTGGCCCTCACTAACACGGCTTCTCCTCCATCCAGTCCTCAT | Yes |
| A1472Y | CTTTATGACGGTCTGTCGAGCTTGGCCCCCACTAACACGGCTTCTCCTCCATCCAGTCCTCAT | Yes |
| Del LAD | -----------GGTCTGTCGAGCTTGGCCCCCACTAACACGGCTTCTCCTCCATCCASTCCTCAT | Yes |

These results suggest that calpains recognize and cleave a structural domain instead of a precise amino acids sequence. For instance, injured HEK cells transfected with dysferlin transcript 11 delN1 (deletion of exon 40a first seven aa) and transcript 11 delN2 (deletion of exon 40a second quarter) present a weaker mini-dysferlin band compared to injured HEK cells transfected with dysferlin transcript 11 (Figure 3B). Inhibitor of calpain 1 and 2 prevents calpain cleavage in DYSF exon 40a (data not shown), confirming that cleavage is due to calpain activity. Altogether these experiments demonstrate that dysferlin is cleaved in exon40a by calpains (1 and 2) in the central region of this sequence. Furthermore, the deletions made within exon 40a indicate that calpains recognize a sequence towards the N-terminal part of the exon. This minimal sequence is composed of the first 11 amino acids of the polypeptide encoded by exon 40a.

### 2.2. Dysferlin localization in muscle cell

In 2013, Lek et al ("Calpains, cleaved Mini-DysferlinC72 and L-type channels underpin calcium-dependent muscle membrane repair", J. Neurosc., March 20, 2013, 33(12):5085-94) demonstrated that during membrane repair process, dysferlin cleavage by calpains within exon 40a releasing a C-terminal fragment named mini- dysferlin which is enriched at membrane injury sites. To explore the fate of this mini-dysferlin, murine myoblasts were transfected with dysferlin transcript 1 (major transcript) or dysferlin transcript 11 (transcript containing exon 40a) to monitor the dysferlin localization after membrane injury (Figures 1B and 1C). Lesions were generated by glass beads injuries. Dysferlin labelling was done using GFP staining for the N-terminal domain and Hamlet-1 antibody recognizing a C-terminal epitope. In uninjured myoblasts transfected with dysferlin transcript 1 or dysferlin transcript 11, no difference in localization between the N-terminal and C-terminal parts of dysferlin were detected (Figure 1B). In injured myoblasts transfected with dysferlin transcript1, the same co-localization of the N and C-terminal parts were observed (Figure 1C). In contrast to what was observed, in injured myoblasts transfected with dysferlin transcript 11, the C- terminal part of dysferlin revealed by the Hamlet-1 antibody was clearly enriched at the membrane level and was no longer uniformly co-located with the N-terminal part (Figure 1C). This result supports the fact that when cleaved the C-terminal fragment of dysferlin is recruited to membrane injury site.

### 2.3. Dysferlin transcript 11 is essential in muscle cell membrane repair

To demonstrate the importance of the dysferlin transcript 11, a membrane repair assay was performed on wild-type human myoblasts (WT), dysferlin-null human myoblasts (DYSF-null) and transfected dysferlin-null human myoblasts (DYSF-null + mCherry-dysferlin1, DYSF-null+ mCherry-dysferlin11 and DYSF-null + mCherry-dysferlin1 + mCherry-dysferlin11) in presence of calcium and membrane-impermeable dye FM 1-43 (Figure 3A). Laser membrane injuries on wild-type myoblasts lead to a FM dye entry that cease within a minute of injury whereas it continued steadily after 5 min in dysferlin-null myoblasts. When dysferlin transcript 1 is expressed in dysferlin-null myoblasts, we can see an important FM dye entry during injury, nevertheless it is slowing down after a few minutes. On the other hand, dysferlin-null myoblasts expressing dysferlin transcript 11 that are injured show a slight FM dye entry that cease within a minute, such as wild-type myoblasts. A significative difference (p < 0.01) was observed between un-transfected dysferlin-null myoblasts and dysferlin-null myoblasts expressing dysferlin transcript 11. These results indicate that dysferlin transcript 11 is essential in the muscle cell membrane repair mechanism after laser-induced injury.

### 2.4. Dysferlin transcript 11 participates in the protection of membrane myoblasts from mechanical stress induced by osmotic shock

The role of dysferlin transcript 11 in the protection of myoblast membrane was explored by an osmotic shock assay on wild-type human myoblasts (WT), dysferlin-null human myoblasts (DYSF-null) and transfected dysferlin-null human myoblasts (DYSF-null + GFP-dysferlin1, DYSF-null + GFP-dysferlin11 and DYSF-null + GFP-dysferlin1 + GFP-dysferlin11) (Figure 4A). Cells were placed in a hypotonic solution. This hypo-osmotic shock leads to cell swelling which induces mechanical stresses on the cell membrane. To monitor the integrity of the cell membrane, we have observed the plasmids GFP fluorescence that leaks out of the cells when the plasma membrane is damaged. The percentage of cell survival for wild- type myoblasts during hypo-osmotic shock decreased slightly the first fifteen minutes but it seems to stabilize after that (86% of cell survival percentage after 20 min of hypo-osmotic shock). On the contrary, hypo-osmotic shock on dysferlin-null myoblasts induces a decrease of cell survival percentage not stabilized after 20min (68% of cell survival percentage after 20 min of hypo-osmotic shock). When dysferlin transcript 1 or dysferlin transcript 11 was expressed in dysferlin-null myoblasts, we detected a slight decrease of cell survival percentage during hypo- osmotic shock, similar to wild-type myoblasts (90% of cell survival percentage after 20 min of hypo-osmotic shock). These observations showed that dysferlin transcript 1 and dysferlin transcript 11 may protect myoblasts from mechanical stresses induced by hypo-osmotic shocks.

### 2.5. Dysferlin transcript 11 participates in protein vesicle trafficking

Several publications have involved dysferlin in protein vesicle trafficking. To explore the role of dysferlin transcript 11 in protein vesicle trafficking, a transferrin assay was performed on wild-type human myoblasts (WT), dysferlin-null human myoblasts (DYSF-null) and transfected dysferlin-null human myoblasts (DYSF-null + mCherry-dysferlin1, DYSF-null + mCherry-dysferlin11 and DYSF-null + mCherry-dysferlin1 + mCherry-dysferlin11) (Figure 7). Transferrin is a molecule rapidly internalized into cells via the transferrin receptor. After internalization, transferrin is recycled to the plasma membrane. To explore the internalization process, myoblasts were incubated for 30 min with transferrin coupled to the fluorophore Alexa-488. The recycling function was also evaluated by incubating myoblasts for 30 min with transferrin and then incubating myoblasts for 30 min in transferrin-free medium. For each cell, we quantified the intensity of transferrin. Internalization function (pulse) compared with recycling function (pulse-chase) showed a significative difference for wild-type myoblasts compared to dysferlin-null myoblasts. The difference between fluorescence levels for pulse and pulse-chase were greater when comparing control myoblasts (-1.25 effect size, Cohen's coefficient) to dysferlin-deficient myoblasts (-0.2 effect size). In dysferlin-null myoblasts expressing dysferlin transcript 1 or dysferlin transcript 11, significant difference between pulse and pulse-chase were observed (-0.898 effect size for dysferlin transcript 1 and -1.53 effect size for dysferlin transcript 11). These observations indicated that both transcripts participated in transferrin trafficking in muscle cell.

This study demonstrates the importance of the dysferlin11 transcript which contains the alternative exon 40a. Indeed, membrane injuries of muscle cells induce the establishment of a repair mechanism that causes dysferlin cleavage by calpains within exon 40a, releasing a C-terminal fragment named mini-dysferlin.

Cell scrape injury and western blot analysis performed in this study show that cleavage in *DYSF* exon 40a is carried out by calpains 1 and/or 2. Several studies expose the predictions of calpains cleavage, but there seems to be no explicit rule for calpain specificity. Actually, amino acid sequences around cleavage sites by calpain are very diverse. It is therefore difficult to precisely determine the location of cleavage sites by calpain. Our results demonstrate that partial deletion of exon 40a decrease calpain cleavage (delC, delN1 and delN2) and deletion of exon 40a first half prevent calpain cleavage (deIN). Therefore, the entire sequence of exon 40a is important for the cleavage of dysferlin by calpains but N-terminal part of this alternative exon is more important than others.

Results show that substitutions and small deletions in DYSF exon 40a do not prevent calpain cleavage. Thus, it has been observed that amino acid substitution or deletion of one to seven amino acids do not prevent dysferlin cleavage by calpains. Only the deletion of the twelve first amino acids of DYSF exon 40a avoids cleavage of dysferlin. These observations confirm that calpains recognize a large amino acid sequence to cleave dysferlin.

On the other hand, myoferlin, which is a member of ferlin protein family, is also cleaved by calpains within an alternative exon, releasing a C-terminal fragment that has 62.82% amino acid identity with the mini-dysferlin. There seems to be an evolutionary preservation of enzymatic cleavage of both dysferlin and myoferlin. Indeed, DYSF alternative exon 40a is conserved in the majority of mammals.

Given the importance of the region coded by DYSF exon 40a (site of dysferlin cleavage by calpains during repair of the muscle cell membrane), dysferlin transcript 11 must have a particular importance in the functions of the muscle cell. Actually, our results indicate that this transcript is essential to the reparation of the muscle cell membrane. Main transcript of dysferlin (transcript 1) seems to be little involved in the repair mechanism of the muscle cell membrane when the latter suffer a laser-injury. In that case, only dysferlin transcript 11 which contains exon 40a makes it possible to repair the muscle cell membrane injured with a laser. These results are in line with the work of Lek and her team in 2013 that showed dysferlin cleavage at exon 40a by calpains and accumulation of mini-dysferlin at lesion site during repair of the muscle cell membrane.

Otherwise, dysferlin is involved in other muscle cell functions. Our results confirm this previous work: dysferlin-null myoblasts are more likely to die from hypo-osmotic shock unlike wild-type myoblasts. Our results also show that restoration of dysferlin transcript 1 and/or dysferlin transcript 11 seem to increase the cells resistance to mechanical stress induced by osmotic shock. This observation indicates that alternative exon 40a is not essential for muscle cell membrane protection, but dysferlin transcript 11 is able to accomplish it.

Moreover, dysferlin is involved in protein vesicle trafficking. It is known that dysferlin-null myoblasts accumulate transferrin and transferrin endocytic recycling is delayed in these cells. The present data show equally an abnormal vesicular trafficking in dysferlin-null myoblasts compared to wild-type myoblasts and dysferlin-null myoblasts with a dysferlin restoration. These data demonstrate that dysferlin-null myoblasts have a lower transferrin accumulation than wild-type myoblasts, and that they do not show a significant difference between endocytosis function (pulse) and recycling function (pulse-chase). When dysferlin transcript 1 and/or dysferlin transcript 11 is restored in dysferlin-null myoblasts, we show again a significant difference between endocytosis and recycling functions which demonstrates a restoration of vesicular trafficking. These two dysferlin transcripts seem to participate in transferrin trafficking in muscle cell. This observation indicates, as before, that alternative exon 40a is not essential for protein vesicle trafficking, but dysferlin transcript 11 is able to accomplish it.

All of these results indicate that dysferlin transcript 1 but also dysferlin transcript 11 have an important place in the functions of the muscle cell. In terms of therapy, these findings suggest that dysferlin transcript containing exon 40a should be restored in patients with dysferlinopathy. Currently, all therapeutic studies conducted so far restore only the main transcript of dysferlin (transcript 1). These studies show promising results: high levels of dysferlin expression, improvement of the histological aspect of the muscle, reduction of necrotic fibers and global improvement in locomotor activity. Regarding the muscle membrane repair function, the improvements obtained do not restore the function to the same level as seen in the wild-type individuals. It would be interesting to restore dysferlin transcript 11 in models of dysferlinopathies to obtain better results in the repair function of the muscle cell membrane.

Among these SNPs, only two missense polymorphisms were predicted pathogenic by UMD-Predictor: c.4704C>T and c.4707G>T. Therefore, we carried out constructions in which we induced a substitution of the amino acids concerned by these two SNPs predicted pathogenic. Cell scrape injury and western blot analysis performed with these two constructions show that amino acid substitutions at these positions do not avoid the cleavage of dysferlin by calpains during the repair mechanism of muscle cell membrane. Moreover, our results indicate that substitutions and small deletions in *DYSF* exon 40a do not prevent calpain cleavage. These observations indicate that variants in exon 40a do not prevent the formation of mini-dysferlin, which has a major role in membrane repair mechanism. Knowing this, we can assume that variants in the alternative exon 40a do not lead to membrane repair defect. Nevertheless, as transcript containing exon 40a are strongly expressed in liver, lung, placenta and pancreas (between 40 and 60%), variants present in this exon could lead to non-muscular deleterious phenotype.

These data demonstrate the importance of the dysferlin transcript 11, which contains the alternative exon 40a, in muscle cell functions and more particularly in the reparation mechanism of the muscle cell membrane. During this mechanism, calpains 1 and/or 2 cleave dysferlin in the first part of *DYSF* exon 40a, releasing a C-terminal fragment named mini-dysferlin. Our results show that dysferlin transcript 11 is essential in muscle cell membrane repair, but it also participates in muscle cell membrane protection and protein vesicle trafficking. All these results indicate that dysferlin transcript 11 have an important place in the functions of the muscle cell. In terms of therapy, these findings suggest that dysferlin transcript containing exon 40a should be restore in patients with dysferlinopathy.

### EXAMPLE 2: Midi-dysferlin for gene therapy

The inventors designed a midi-dysferlin protein, comprising at least, from its Nt extremity to its Ct extremity:
- A C2A domain, or a functional equivalent thereof
- A polypeptide encoded by exon40a, a functional fragment thereof or a functional derivative thereof
- A C2F domain, or a functional equivalent thereof
- A C2G domain, or a functional equivalent thereof
- A transmembrane domain (TM).

Midi-dysferlin (SEQ ID N°21) was transfected into dysferlin-null myoblasts. Western blot performed with proteins from wild-type myoblasts (WT, C25), dysferlin-null myoblasts (DYSF-null, AB320) and transfected dysferlin-null myoblasts (DYSF-null + Midi-dysferlin), wherein Hamlet antibodies were used to detect dysferlin and midi-dysferlin, showed the expression of midi-dysferlin in transfected dysferlin-null myoblasts (Figures 8A and 8B).

Experimental data with midi-dysferlin, which were performed in conditions similar to those described in example 1 with dysferlin, show that:
- Midi-dysferlin allows a complete restoration of muscle-cell membrane repair (Figures 7A and 7B).
- Midi-dysferlin allows a partial restoration of myoblasts membrane protection from mechanical stress induced by osmotic shock (Figure 8).
- Midi-dysferlin participates in protein vesicle trafficking (Figure 9).

Experimental data show that a "midi-dysferlin" protein, which comprises exon 40a, can be cleaved by calpain and is able to repair muscle membrane lesion, to restore myoblasts membrane protection from mechanical stress induced by osmotic shock and to participate in protein vesicle trafficking. Said "midi-dysferlin" truncated protein is necessary and sufficient to trigger membrane repair pathway after injury.

## Claims

1. An isolated nucleic acid sequence comprising, or consisting of, from its 5' to 3' extremity:
a) a nucleic acid sequence encoding for the dysferlin amino acids N°1 to 301 (SEQ ID N°1), for an amino acid sequence exhibiting at least 80% identity with SEQ ID N°1, or for an amino acid sequence comprising at least 100 amino acids of SEQ ID N°1,
b) a nucleic acid sequence encoding for a polypeptide comprising a cleavage site by calpain, and
c) a nucleic acid sequence encoding for the dysferlin C2F, C2G and TM domains (SEQ ID N°2), for an amino acid sequence exhibiting at least 80% identity with said SEQ ID N°2 or for an amino acid sequence comprising at least 100 amino acids of SEQ ID N°2.

2. An isolated nucleic acid sequence according to claim 1, wherein said nucleic acid sequence encoding for at least 100 amino acids of the dysferlin N-terminal amino acids N°1 to 301 encodes for the amino acid sequence of C2A domain (SEQ ID N°3), or for an amino acid sequence exhibiting at least 80% identity with SEQ ID N°3.

3. An isolated nucleic acid sequence according to claim 1 or 2, wherein said nucleic acid sequence encodes for a polypeptide comprising a cleavage site by calpain, said polypeptide comprising or consisting of the amino acid sequence SEQ ID N°4, a functional fragment thereof comprising at least a sequence SEQ ID N°5, or an amino acid sequence exhibiting at least 80% identity with SEQ ID N°6.

4. An isolated nucleic acid sequence according to claim 1 or 2, wherein said nucleic acid sequence encoding for a cleavage site by calpain is nucleic acid sequence SEQ ID N°A, corresponding to dysferlin exon 40a, a nucleic acid sequence exhibiting at least 80% identity with SEQ ID N°A or a nucleic acid sequence comprising at least 30 nucleotides of SEQ ID N°A.

5. An isolated nucleic acid sequence according to any of the preceding claims, comprising, from its 5' extremity to its 3' extremity:
i. a nucleic acid sequence encoding for the dysferlin C2A domain (SEQ ID N°3),
ii. a nucleic acid sequence corresponding to exon 40a (SEQ ID N°A), and
iii. a nucleic acid sequence encoding for the dysferlin C2F, C2G and TM domains (SEQ ID N°2).

6. An isolated nucleic acid sequence according to any of the preceding claims which is SEQ ID N°B, or a nucleic acid sequence exhibiting at least 80 % identity with SEQ ID N°B.

7. A recombinant vector comprising an isolated nucleic acid sequence according to any one of preceding claims.

8. A recombinant vector comprising an isolated nucleic acid sequence according to any of the preceding claims, which is a recombinant viral vector, said vector being preferably chosen among:

9. A pharmaceutical composition comprising a recombinant vector according to any of the preceding claims and a therapeutically acceptable carrier, diluent or excipient.

10. Use of a recombinant vector according to any one of claims Y to YY as a drug for the treatment of a dysferlinopathy.

11. Use according to the preceding claim, wherein said dysferlinopathy is a myopathy chosen among: Miyoshi myopathy (MM) and limb girdle muscular dystrophy type 2B (LGMD2B or LGMDR2).


SEQUENCE LISTING

<110>  UNIVERSITE D'AIX MARSEILLE
       INSERM (Institut National de la Santé et de la Recherche
       Médicale)
       Bartoli, Marc
       Courrier, Sébastien
       Ballouhey, Océane

<120>  COMPOSITION FOR TREATING DYSFERLINOPATHY

<130>  IOBM21SSEDYS

<160>  32

<170>  PatentIn version 3.5

<210>  1
<211>  301
<212>  PRT
<213>  Homo sapiens

<400>  1

Met Leu Arg Val Phe Ile Leu Tyr Ala Glu Asn Val His Thr Pro Asp
1               5                   10                  15

Thr Asp Ile Ser Asp Ala Tyr Cys Ser Ala Val Phe Ala Gly Val Lys
            20                  25                  30

Lys Arg Thr Lys Val Ile Lys Asn Ser Val Asn Pro Val Trp Asn Glu
        35                  40                  45

Gly Phe Glu Trp Asp Leu Lys Gly Ile Pro Leu Asp Gln Gly Ser Glu
    50                  55                  60

Leu His Val Val Val Lys Asp His Glu Thr Met Gly Arg Asn Arg Phe
65                  70                  75                  80

Leu Gly Glu Ala Lys Val Pro Leu Arg Glu Val Leu Ala Thr Pro Ser
                85                  90                  95

Leu Ser Ala Ser Phe Asn Ala Pro Leu Leu Asp Thr Lys Lys Gln Pro
            100                 105                 110

Thr Gly Ala Ser Leu Val Leu Gln Val Ser Tyr Thr Pro Leu Pro Gly
        115                 120                 125

Ala Val Pro Leu Phe Pro Pro Pro Thr Pro Leu Glu Pro Ser Pro Thr
    130                 135                 140

Leu Pro Asp Leu Asp Val Val Ala Asp Thr Gly Gly Glu Glu Asp Thr
145                 150                 155                 160

Glu Asp Gln Gly Leu Thr Gly Asp Glu Ala Glu Pro Phe Leu Asp Gln
                165                 170                 175

Ser Gly Gly Pro Gly Ala Pro Thr Thr Pro Arg Lys Leu Pro Ser Arg
            180                 185                 190

Pro Pro Pro His Tyr Pro Gly Ile Lys Arg Lys Arg Ser Ala Pro Thr
        195                 200                 205

Ser Arg Lys Leu Leu Ser Asp Lys Pro Gln Asp Phe Gln Ile Arg Val
    210                 215                 220

Gln Val Ile Glu Gly Arg Gln Leu Pro Gly Val Asn Ile Lys Pro Val
225                 230                 235                 240

Val Lys Val Thr Ala Ala Gly Gln Thr Lys Arg Thr Arg Ile His Lys
                245                 250                 255

Gly Asn Ser Pro Leu Phe Asn Glu Thr Leu Phe Phe Asn Leu Phe Asp
            260                 265                 270

Ser Pro Gly Glu Leu Phe Asp Glu Pro Ile Phe Ile Thr Val Val Asp
        275                 280                 285

Ser Arg Ser Leu Arg Thr Asp Ala Leu Leu Gly Glu Phe
    290                 295                 300

<210>  2
<211>  517
<212>  PRT
<213>  Homo sapiens

<400>  2

Pro Arg Gln Phe His Gln Leu Ala Ala Gln Gly Pro Gln Glu Cys Leu
1               5                   10                  15

Val Arg Ile Tyr Ile Val Arg Ala Phe Gly Leu Gln Pro Lys Asp Pro
            20                  25                  30

Asn Gly Lys Cys Asp Pro Tyr Ile Lys Ile Ser Ile Gly Lys Lys Ser
        35                  40                  45

Val Ser Asp Gln Asp Asn Tyr Ile Pro Cys Thr Leu Glu Pro Val Phe
    50                  55                  60

Gly Lys Met Phe Glu Leu Thr Cys Thr Leu Pro Leu Glu Lys Asp Leu
65                  70                  75                  80

Lys Ile Thr Leu Tyr Asp Tyr Asp Leu Leu Ser Lys Asp Glu Lys Ile
                85                  90                  95

Gly Glu Thr Val Val Asp Leu Glu Asn Arg Leu Leu Ser Lys Phe Gly
            100                 105                 110

Ala Arg Cys Gly Leu Pro Gln Thr Tyr Cys Val Ser Gly Pro Asn Gln
        115                 120                 125

Trp Arg Asp Gln Leu Arg Pro Ser Gln Leu Leu His Leu Phe Cys Gln
    130                 135                 140

Gln His Arg Val Lys Ala Pro Val Tyr Arg Thr Asp Arg Val Met Phe
145                 150                 155                 160

Gln Asp Lys Glu Tyr Ser Ile Glu Glu Ile Glu Ala Gly Arg Ile Pro
                165                 170                 175

Asn Pro His Leu Gly Pro Val Glu Glu Arg Leu Ala Leu His Val Leu
            180                 185                 190

Gln Gln Gln Gly Leu Val Pro Glu His Val Glu Ser Arg Pro Leu Tyr
        195                 200                 205

Ser Pro Leu Gln Pro Asp Ile Glu Gln Gly Lys Leu Gln Met Trp Val
    210                 215                 220

Asp Leu Phe Pro Lys Ala Leu Gly Arg Pro Gly Pro Pro Phe Asn Ile
225                 230                 235                 240

Thr Pro Arg Arg Ala Arg Arg Phe Phe Leu Arg Cys Ile Ile Trp Asn
                245                 250                 255

Thr Arg Asp Val Ile Leu Asp Asp Leu Ser Leu Thr Gly Glu Lys Met
            260                 265                 270

Ser Asp Ile Tyr Val Lys Gly Trp Met Ile Gly Phe Glu Glu His Lys
        275                 280                 285

Gln Lys Thr Asp Val His Tyr Arg Ser Leu Gly Gly Glu Gly Asn Phe
    290                 295                 300

Asn Trp Arg Phe Ile Phe Pro Phe Asp Tyr Leu Pro Ala Glu Gln Val
305                 310                 315                 320

Cys Thr Ile Ala Lys Lys Asp Ala Phe Trp Arg Leu Asp Lys Thr Glu
                325                 330                 335

Ser Lys Ile Pro Ala Arg Val Val Phe Gln Ile Trp Asp Asn Asp Lys
            340                 345                 350

Phe Ser Phe Asp Asp Phe Leu Gly Ser Leu Gln Leu Asp Leu Asn Arg
        355                 360                 365

Met Pro Lys Pro Ala Lys Thr Ala Lys Lys Cys Ser Leu Asp Gln Leu
    370                 375                 380

Asp Asp Ala Phe His Pro Glu Trp Phe Val Ser Leu Phe Glu Gln Lys
385                 390                 395                 400

Thr Val Lys Gly Trp Trp Pro Cys Val Ala Glu Glu Gly Glu Lys Lys
                405                 410                 415

Ile Leu Ala Gly Lys Leu Glu Met Thr Leu Glu Ile Val Ala Glu Ser
            420                 425                 430

Glu His Glu Glu Arg Pro Ala Gly Gln Gly Arg Asp Glu Pro Asn Met
        435                 440                 445

Asn Pro Lys Leu Glu Asp Pro Arg Arg Pro Asp Thr Ser Phe Leu Trp
    450                 455                 460

Phe Thr Ser Pro Tyr Lys Thr Met Lys Phe Ile Leu Trp Arg Arg Phe
465                 470                 475                 480

Arg Trp Ala Ile Ile Leu Phe Ile Ile Leu Phe Ile Leu Leu Leu Phe
                485                 490                 495

Leu Ala Ile Phe Ile Tyr Ala Phe Pro Asn Tyr Ala Ala Met Lys Leu
            500                 505                 510

Val Lys Pro Phe Ser
        515

<210>  3
<211>  127
<212>  PRT
<213>  Homo sapiens

<400>  3

Met Leu Arg Val Phe Ile Leu Tyr Ala Glu Asn Val His Thr Pro Asp
1               5                   10                  15

Thr Asp Ile Ser Asp Ala Tyr Cys Ser Ala Val Phe Ala Gly Val Lys
            20                  25                  30

Lys Arg Thr Lys Val Ile Lys Asn Ser Val Asn Pro Val Trp Asn Glu
        35                  40                  45

Gly Phe Glu Trp Asp Leu Lys Gly Ile Pro Leu Asp Gln Gly Ser Glu
    50                  55                  60

Leu His Val Val Val Lys Asp His Glu Thr Met Gly Arg Asn Arg Phe
65                  70                  75                  80

Leu Gly Glu Ala Lys Val Pro Leu Arg Glu Val Leu Ala Thr Pro Ser
                85                  90                  95

Leu Ser Ala Ser Phe Asn Ala Pro Leu Leu Asp Thr Lys Lys Gln Pro
            100                 105                 110

Thr Gly Ala Ser Leu Val Leu Gln Val Ser Tyr Thr Pro Leu Pro
        115                 120                 125

<210>  4
<211>  21
<212>  PRT
<213>  Homo sapiens

<400>  4

Leu Ala Asp Gly Leu Ser Ser Leu Ala Pro Thr Asn Thr Ala Ser Pro
1               5                   10                  15

Pro Ser Ser Pro His
            20

<210>  5
<211>  12
<212>  PRT
<213>  Homo sapiens

<400>  5

Leu Ala Asp Gly Leu Ser Ser Leu Ala Pro Thr Asn
1               5                   10

<210>  6
<211>  8
<212>  PRT
<213>  Artificial

<220>
<223>  Amino acid sequence from spectrin, subject to calpain cleavage

<400>  6

Glu Glu Val Tyr Gly Met Met Lys

1               5

<210>  7
<211>  809
<212>  PRT
<213>  Artificial

<220>
<223>  Midi-dysferline comprises dysferlin Nt domain, polypeptide
       encoded by exon 40a and dysferlin Ct domain

<400>  7

Met Leu Arg Val Phe Ile Leu Tyr Ala Glu Asn Val His Thr Pro Asp
1               5                   10                  15

Thr Asp Ile Ser Asp Ala Tyr Cys Ser Ala Val Phe Ala Gly Val Lys
            20                  25                  30

Lys Arg Thr Lys Val Ile Lys Asn Ser Val Asn Pro Val Trp Asn Glu
        35                  40                  45

Gly Phe Glu Trp Asp Leu Lys Gly Ile Pro Leu Asp Gln Gly Ser Glu
    50                  55                  60

Leu His Val Val Val Lys Asp His Glu Thr Met Gly Arg Asn Arg Phe
65                  70                  75                  80

Leu Gly Glu Ala Lys Val Pro Leu Arg Glu Val Leu Ala Thr Pro Ser
                85                  90                  95

Leu Ser Ala Ser Phe Asn Ala Pro Leu Leu Asp Thr Lys Lys Gln Pro
            100                 105                 110

Thr Gly Ala Ser Leu Val Leu Gln Val Ser Tyr Thr Pro Leu Pro Gly
        115                 120                 125

Ala Val Pro Leu Phe Pro Pro Pro Thr Pro Leu Glu Pro Ser Pro Thr
    130                 135                 140

Leu Pro Asp Leu Asp Val Val Ala Asp Asp Val Ser Leu Leu Ser Pro
145                 150                 155                 160

Gly Glu Asp Val Leu Ile Asp Ile Asp Asp Lys Glu Pro Leu Ile Pro
                165                 170                 175

Ile Gln Leu Ala Asp Gly Leu Ser Ser Leu Ala Pro Thr Asn Thr Ala
            180                 185                 190

Ser Pro Pro Ser Ser Pro His Glu Glu Glu Phe Ile Asp Trp Trp Ser

195                 200                 205

Lys Phe Phe Ala Ser Ile Gly Glu Arg Glu Lys Cys Gly Ser Tyr Leu
    210                 215                 220

Glu Lys Asp Phe Asp Thr Leu Lys Val Tyr Asp Thr Gln Leu Glu Asn
225                 230                 235                 240

Val Glu Ala Phe Glu Gly Leu Ser Asp Phe Cys Asn Thr Phe Lys Leu
                245                 250                 255

Tyr Arg Gly Lys Thr Gln Glu Glu Thr Glu Asp Pro Ser Val Ile Gly
            260                 265                 270

Glu Phe Lys Gly Leu Phe Lys Ile Tyr Pro Leu Pro Glu Asp Pro Ala
        275                 280                 285

Ile Pro Met Pro Pro Arg Gln Phe His Gln Leu Ala Ala Gln Gly Pro
    290                 295                 300

Gln Glu Cys Leu Val Arg Ile Tyr Ile Val Arg Ala Phe Gly Leu Gln
305                 310                 315                 320

Pro Lys Asp Pro Asn Gly Lys Cys Asp Pro Tyr Ile Lys Ile Ser Ile
                325                 330                 335

Gly Lys Lys Ser Val Ser Asp Gln Asp Asn Tyr Ile Pro Cys Thr Leu
            340                 345                 350

Glu Pro Val Phe Gly Lys Met Phe Glu Leu Thr Cys Thr Leu Pro Leu
        355                 360                 365

Glu Lys Asp Leu Lys Ile Thr Leu Tyr Asp Tyr Asp Leu Leu Ser Lys
    370                 375                 380

Asp Glu Lys Ile Gly Glu Thr Val Val Asp Leu Glu Asn Arg Leu Leu
385                 390                 395                 400

Ser Lys Phe Gly Ala Arg Cys Gly Leu Pro Gln Thr Tyr Cys Val Ser
                405                 410                 415

Gly Pro Asn Gln Trp Arg Asp Gln Leu Arg Pro Ser Gln Leu Leu His
            420                 425                 430

Leu Phe Cys Gln Gln His Arg Val Lys Ala Pro Val Tyr Arg Thr Asp
        435                 440                 445

Arg Val Met Phe Gln Asp Lys Glu Tyr Ser Ile Glu Glu Ile Glu Ala
    450                 455                 460

Gly Arg Ile Pro Asn Pro His Leu Gly Pro Val Glu Glu Arg Leu Ala
465                 470                 475                 480

Leu His Val Leu Gln Gln Gln Gly Leu Val Pro Glu His Val Glu Ser
                485                 490                 495

Arg Pro Leu Tyr Ser Pro Leu Gln Pro Asp Ile Glu Gln Gly Lys Leu
            500                 505                 510

Gln Met Trp Val Asp Leu Phe Pro Lys Ala Leu Gly Arg Pro Gly Pro
        515                 520                 525

Pro Phe Asn Ile Thr Pro Arg Arg Ala Arg Arg Phe Phe Leu Arg Cys
    530                 535                 540

Ile Ile Trp Asn Thr Arg Asp Val Ile Leu Asp Asp Leu Ser Leu Thr
545                 550                 555                 560

Gly Glu Lys Met Ser Asp Ile Tyr Val Lys Gly Trp Met Ile Gly Phe
                565                 570                 575

Glu Glu His Lys Gln Lys Thr Asp Val His Tyr Arg Ser Leu Gly Gly
            580                 585                 590

Glu Gly Asn Phe Asn Trp Arg Phe Ile Phe Pro Phe Asp Tyr Leu Pro
        595                 600                 605

Ala Glu Gln Val Cys Thr Ile Ala Lys Lys Asp Ala Phe Trp Arg Leu
    610                 615                 620

Asp Lys Thr Glu Ser Lys Ile Pro Ala Arg Val Val Phe Gln Ile Trp
625                 630                 635                 640

Asp Asn Asp Lys Phe Ser Phe Asp Asp Phe Leu Gly Ser Leu Gln Leu
                645                 650                 655

Asp Leu Asn Arg Met Pro Lys Pro Ala Lys Thr Ala Lys Lys Cys Ser
            660                 665                 670

Leu Asp Gln Leu Asp Asp Ala Phe His Pro Glu Trp Phe Val Ser Leu
        675                 680                 685

Phe Glu Gln Lys Thr Val Lys Gly Trp Trp Pro Cys Val Ala Glu Glu
    690                 695                 700

Gly Glu Lys Lys Ile Leu Ala Gly Lys Leu Glu Met Thr Leu Glu Ile
705                 710                 715                 720

Val Ala Glu Ser Glu His Glu Glu Arg Pro Ala Gly Gln Gly Arg Asp
                725                 730                 735

Glu Pro Asn Met Asn Pro Lys Leu Glu Asp Pro Arg Arg Pro Asp Thr
            740                 745                 750

Ser Phe Leu Trp Phe Thr Ser Pro Tyr Lys Thr Met Lys Phe Ile Leu
        755                 760                 765

Trp Arg Arg Phe Arg Trp Ala Ile Ile Leu Phe Ile Ile Leu Phe Ile
    770                 775                 780

Leu Leu Leu Phe Leu Ala Ile Phe Ile Tyr Ala Phe Pro Asn Tyr Ala
785                 790                 795                 800

Ala Met Lys Leu Val Lys Pro Phe Ser
                805

<210>  8
<211>  21
<212>  PRT
<213>  Artificial

<220>
<223>  Mutated polypeptide  S1485A and P1487A

<400>  8

Leu Ala Asp Gly Leu Ser Ser Leu Ala Pro Thr Asn Thr Ala Ala Pro
1               5                   10                  15

Ala Ser Ser Pro His
            20

<210>  9
<211>  21
<212>  PRT
<213>  Artificial

<220>
<223>  Mutated polypeptide S1485A ; P1487A ; S1489G

<400>  9

Leu Ala Asp Gly Leu Ser Ser Leu Ala Pro Thr Asn Thr Ala Ala Pro
1               5                   10                  15

Ala Ser Gly Pro His
            20

<210>  10
<211>  21
<212>  PRT
<213>  Artificial

<220>
<223>  Mutated polypeptide S1476Y

<400>  10

Leu Ala Asp Gly Leu Tyr Ser Leu Ala Pro Thr Asn Thr Ala Ser Pro
1               5                   10                  15

Pro Ser Ser Pro His
            20

<210>  11
<211>  21
<212>  PRT
<213>  Artificial

<220>
<223>  Mutated polypeptide S1476A ; S1477G

<400>  11

Leu Ala Asp Gly Leu Ala Gly Leu Ala Pro Thr Asn Thr Ala Ser Pro
1               5                   10                  15

Pro Ser Ser Pro His
            20

<210>  12
<211>  21
<212>  PRT
<213>  Artificial

<220>
<223>  Mutated polypeptide T1481A

<400>  12

Leu Ala Asp Gly Leu Ser Ser Leu Ala Pro Ala Asn Thr Ala Ser Pro
1               5                   10                  15

Pro Ser Ser Pro His
            20

<210>  13
<211>  21
<212>  PRT
<213>  Artificial

<220>
<223>  Mutated polypeptide T1483K

<400>  13

Leu Ala Asp Gly Leu Ser Ser Leu Ala Pro Thr Asn Lys Ala Ser Pro
1               5                   10                  15

Pro Ser Ser Pro His
            20

<210>  14
<211>  17
<212>  PRT
<213>  Artificial

<220>
<223>  Mutated polypeptide "Del PTNT"

<400>  14

Leu Ala Asp Gly Leu Ser Ser Leu Ala Ala Ser Pro Pro Ser Ser Pro
1               5                   10                  15

His

<210>  15
<211>  20
<212>  PRT
<213>  Artificial

<220>
<223>  Mutated polypeptide Del P1480

<400>  15

Leu Ala Asp Gly Leu Ser Ser Leu Ala Thr Asn Thr Ala Ser Pro Pro
1               5                   10                  15

Ser Ser Pro His
            20

<210>  16
<211>  21
<212>  PRT
<213>  Artificial

<220>
<223>  Mutated polypeptide P1480S

<400>  16

Leu Ala Asp Gly Leu Ser Ser Leu Ala Leu Thr Asn Thr Ala Ser Pro
1               5                   10                  15

Pro Ser Ser Pro His
            20

<210>  17
<211>  21
<212>  PRT
<213>  Artificial

<220>
<223>  Mutated polypeptide A1472Y

<400>  17

Leu Tyr Asp Gly Leu Ser Ser Leu Ala Pro Thr Asn Thr Ala Ser Pro
1               5                   10                  15

Pro Ser Ser Pro His
            20

<210>  18
<211>  18
<212>  PRT
<213>  Artificial

<220>
<223>  Mutated polypeptide Del LAD

<400>  18

Gly Leu Ser Ser Leu Ala Pro Thr Asn Thr Ala Ser Pro Pro Ser Ser
1               5                   10                  15

Pro His

<210>  19
<211>  63
<212>  DNA
<213>  Homo sapiens

<400>  19
cttgcagacg gtctgtcgag cttggccccc actaacacgg cttctcctcc atccagtcct      60

cat                                                                    63

<210>  20
<211>  2430
<212>  DNA
<213>  Artificial

<220>
<223>  Nucleotide sequence of midi-dysferlin

<400>  20
atgctcagag tcttcattct gtatgccgag aacgtccaca cccccgacac agacattagc      60

gacgcctact gctccgcggt cttcgccggc gtgaaaaaga ggacaaaagt gatcaagaat     120

agcgtgaacc ccgtctggaa tgagggattt gagtgggatc tgaagggcat tcctctggac     180

caaggcagcg agctgcatgt ggtggtcaag gaccacgaga caatgggaag aaataggttt 240

ctgggcgaag ctaaggtgcc tctgagagag gtgctggcta ccccctctct gagcgcctcc 300

ttcaacgctc ctctgctcga cacaaagaag cagcctaccg gcgcttctct ggtgctgcaa 360

gtgagctaca cccctctgcc cggcgctgtg cctctcttcc cccctcctac acctctggag 420

ccttccccta cactgcccga cctcgacgtc gtggccgatg acgtgtctct gctgagcccc 480

ggcgaggatg tgctgatcga cattgacgac aaggaacctc tgatccctat ccaactggcc 540

gatggactgt cctccctcgc ccccaccaat acagcttccc cccctagcag cccccatgag 600

gaggagttca tcgactggtg gagcaaattc ttcgcttcca tcggcgagag agaaaagtgt 660

ggcagctatc tggaaaaaga cttcgatacc ctcaaggtgt atgacaccca gctggagaat 720

gtcgaggctt ttgaaggact gagcgacttc tgcaacacat tcaagctcta tagaggcaaa 780

acccaagagg agaccgaaga cccctccgtg atcggcgagt ttaagggact gttcaagatt 840

taccctctgc ccgaggaccc cgccatccct atgcccccta ggcaattcca tcaactggcc 900

gcccaaggcc cccaagaatg tctggtgagg atttacatcg tcagagcctt cggactgcag 960

cccaaagatc ccaacggcaa gtgcgatccc tatatcaaga tcagcatcgg caaaaagtcc 1020

gtcagcgacc aagacaacta catcccttgc acactggagc ccgtgttcgg caagatgttc 1080

gagctgacat gtacactgcc cctcgaaaag gacctcaaga tcaccctcta tgactacgat 1140

ctgctctcca aagatgaaaa gatcggcgag accgtggtgg atctcgaaaa cagactgctg 1200

tccaagttcg gagctagatg tggactgccc cagacctact gcgtgtccgg acccaaccag 1260

tggagggacc agctgagacc ctcccaactg ctgcatctgt tttgtcagca gcatagagtc 1320

aaagctcccg tgtatagaac cgatagagtc atgttccaag acaaggaata ttccattgag 1380

gagatcgagg ctggaaggat tcctaacccc cacctcggac ccgtggagga gaggctggct 1440

ctgcacgtgc tgcagcaaca aggcctcgtc cccgagcacg tcgagtccag acccctctat 1500

agccccctcc aacccgacat cgagcaaggc aaactccaga tgtgggtgga cctcttcccc 1560

aaagctctcg gcagacccgg cccccccttc aatattaccc ctaggagagc tagaaggttc 1620

tttctgagat gtatcatctg gaatacaaga gacgtgattc tggacgacct cagcctcacc 1680

ggcgagaaga tgtccgacat ctacgtcaag ggctggatga tcggcttcga ggagcacaaa 1740

cagaagaccg atgtgcacta tagatctctg ggcggagaag gcaatttcaa ctggaggttc 1800

atcttccctt tcgattatct ccccgctgag caagtgtgca caatcgccaa gaaggacgcc 1860

ttttggaggc tggacaagac cgagagcaag atccccgcca gagtggtgtt ccagatctgg 1920

gataacgaca agttttcctt cgatgacttt ctgggaagcc tccagctgga tctgaatagg 1980

atgcctaagc ccgccaagac agccaagaag tgcagcctcg atcaactcga tgacgccttc 2040

catcccgagt ggttcgtgag cctcttcgag cagaaaaccg tgaaaggctg gtggccttgt 2100

gtggccgagg aaggagagaa gaagattctg gccggaaagc tggaaatgac actggagatc 2160

gtggctgaat ccgagcatga agagaggccc gccggccaag gaagagacga acccaatatg 2220

aaccccaagc tggaggaccc tagaagaccc gatacatcct ttctgtggtt caccagcccc 2280

tacaagacca tgaaatttat cctctggagg aggtttagat gggctatcat cctctttatc 2340

attctgttca ttctgctgct cttcctcgcc atctttatct acgcctttcc caactacgcc 2400

gctatgaaac tggtcaagcc tttttcctga 2430

<210> 21
<211> 47
<212> DNA
<213> Artificial

<220>
<223> pcDNADysf11delN "Forward"

<400> 21
gcccctcatc cccatccaga acacggcttc tcctccatcc agtcctc 47

<210> 22
<211> 50
<212> DNA
<213> Artificial

<220>
<223> pcDNADysf11delN "Reverse"

<400> 22
gatggaggag aagccgtgtt ctggatgggg atgaggggct ccttgtcatc 50

<210> 23
<211> 46
<212> DNA
<213> Artificial

<220>
<223> pcDNADysf11delC "Forward"

<400> 23
gtcgagcttg gcccccactg aggaagagtt catcgattgg tggagc 46

<210> 24
<211> 48
<212> DNA
<213> Artificial

<220>
<223> Primer pcDNADysf11delC "Reverse"

<400> 24
ccaatcgatg aactcttcct cagtgggggc caagctcgac agaccgtc 48

<210>  25
<211>  45
<212>  DNA
<213>  Artificial

<220>
<223>  pcDNADysf11delN1 "Forward"

<400>  25
cctcatcccc atccagagct tggcccccac taacacggct tctcc                45


<210>  26
<211>  48
<212>  DNA
<213>  Artificial

<220>
<223>  pcDNADysf11delN1 "Reverse"

<400>  26
gttagtgggg gccaagctct ggatggggat gaggggctcc ttgtcatc             48


<210>  27
<211>  45
<212>  DNA
<213>  Artificial

<220>
<223>  pcDNADysf11delN2 "Forward"

<400>  27
gcttgcagac ggtctgtcga acacggcttc tcctccatcc agtcc                45


<210>  28
<211>  45
<212>  DNA
<213>  Artificial

<220>
<223>  pcDNADysf11delN2 "Reverse"

<400>  28
ggaggagaag ccgtgttcga cagaccgtct gcaagctgga tgggg                45


<210>  29
<211>  835
<212>  DNA
<213>  Artificial

<220>
<223>  Nucleotide sequence of GFP used for labelling

<400>  29
atggtgagca agggcgagga gctgttcacc ggggtggtgc ccatcctggt cgagctggac      60

ggcgacgtaa acggccacaa gttcagcgtg tccggcgagg gcgagggcga tgccacctac     120

ggcaagctga ccctgaagtt catctgcacc accggcaagc tgcccgtgcc ctggcccacc     180

ctcgtgacca ccctgaccta cggcgtgcag tgcttcagcc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagca cccagtccgc cctgagcaaa gaccccaacg agaagcgcga tcacatggtc 660

ctgctggagt tcgtgaccgc cgccgggatc actctcggca tggacgagct gtacaagggc 720

cgctcgagtc tagagggccc gcggttcgaa caaaaactca tctcagaaga ggatctgaat 780

atgcataccg gtcatcatca ccatcaccat tgagtttaaa cccgctgatc agcct 835

<210> 30
<211> 711
<212> DNA
<213> Artificial

<220>
<223> Nucleotide sequence for mCherry plasmid

<400> 30
atggtgagca agggcgagga ggataacatg gccatcatca aggagttcat gcgcttcaag 60

gtgcacatgg agggctccgt gaacggccac gagttcgaga tcgagggcga gggcgagggc 120

cgcccctacg agggcaccca gaccgccaag ctgaaggtga ccaagggtgg ccccctgccc 180

ttcgcctggg acatcctgtc ccctcagttc atgtacggct ccaaggccta cgtgaagcac 240

cccgccgaca tccccgacta cttgaagctg tccttccccg agggcttcaa gtgggagcgc 300

gtgatgaact tcgaggacgg cggcgtggtg accgtgaccc aggactcctc cctccaggac 360

ggcgagttca tctacaaggt gaagctgcgc ggcaccaact tcccctccga cggccccgta 420

atgcagaaga agaccatggg ctgggaggcc tcctccgagc ggatgtaccc cgaggacggc 480

gccctgaagg gcgagatcaa gcagaggctg aagctgaagg acggcggcca ctacgacgct 540

gaggtcaaga ccacctacaa ggccaagaag cccgtgcagc tgcccggcgc ctacaacgtc 600

aacatcaagt tggacatcac ctcccacaac gaggactaca ccatcgtgga acagtacgaa 660

cgcgccgagg gccgccactc caccggcggc atggacgagc tgtacaagta g 711

<210> 31
<211> 9596
<212> DNA
<213> Artificial

<220>

<223>  Nucleotide sequence for Dysferlin 1

<400>  31
atgctgaggg tcttcatcct ctatgccgag aacgtccaca cacccgacac cgacatcagc      60

gatgcctact gctccgcggt gtttgcaggg gtgaagaaga gaaccaaagt catcaagaac     120

agcgtgaacc ctgtatggaa tgagggattt gaatgggacc tcaagggcat ccccctggac     180

cagggctctg agcttcatgt ggtggtcaaa gaccatgaga cgatggggag gaacaggttc     240

ctgggggaag ccaaggtccc actccgagag gtcctcgcca cccctagtct gtccgccagc     300

ttcaatgccc ccctgctgga caccaagaag cagcccacag gggcctcgct ggtcctgcag     360

gtgtcctaca caccgctgcc tggagctgtg cccctgttcc cgccccctac tcctctggag     420

ccctccccga ctctgcctga cctggatgta gtggcagaca caggaggaga ggaagacaca     480

gaggaccagg gactcactgg agatgaggcg gagccattcc tggatcaaag cggaggcccg     540

ggggctccca ccaccccaag gaaactacct tcacgtcctc cgccccacta ccccgggatc     600

aaaagaaagc gaagtgcgcc tacatctaga aagctgctgt cagacaaacc gcaggatttc     660

cagatcaggg tccaggtgat cgaggggcgc cagctgccgg gggtgaacat caagcctgtg     720

gtcaaggtta ccgctgcagg gcagaccaag cggacgcgga tccacaaggg aaacagccca     780

ctcttcaatg agactctttt cttcaacttg tttgactctc ctggggagct gtttgatgag     840

cccatcttta tcacggtggt agactctcgt tctctcagga cagatgctct cctcggggag     900

ttccggatgg acgtgggcac catttacaga gagccccggc acgcctatct caggaagtgg     960

ctgctgctct cagaccctga tgacttctct gctggggcca gaggctacct gaaaacaagc    1020

ctttgtgtgc tggggcctgg ggacgaagcg cctctggaga gaaaagaccc ctctgaagac    1080

aaggaggaca ttgaaagcaa cctgctccgg cccacaggcg tagccctgcg aggagcccac    1140

ttctgcctga aggtcttccg ggccgaggac ttgccgcaga tggacgatgc cgtgatggac    1200

aacgtgaaac agatctttgg cttcgagagt aacaagaaga acttggtgga cccctttgtg    1260

gaggtcagct ttgcggggaa aatgctgtgc agcaagatct tggagaagac ggccaaccct    1320

cagtggaacc agaacatcac actgcctgcc atgtttccct ccatgtgcga aaaaatgagg    1380

attcgtatca tagactggga ccgcctgact cacaatgaca tcgtggctac cacctacctg    1440

agtatgtcga aaatctctgc ccctggagga gaaatagaag aggagcctgc aggtgctgtc    1500

aagccttcga aagcctcaga cttggatgac tacctgggct tcctccccac ttttgggccc    1560

tgctacatca acctctatgg cagtcccaga gagttcacag gcttcccaga cccctacaca    1620

gagctcaaca caggcaaggg ggaaggtgtg gcttatcgtg gccggcttct gctctccctg    1680

gagaccaagc tggtggagca cagtgaacag aaggtggagg accttcctgc ggatgacatc    1740

ctccgggtgg agaagtacct taggaggcgc aagtactccc tgtttgcggc cttctactca    1800

gccaccatgc tgcaggatgt ggatgatgcc atccagtttg aggtcagcat cgggaactac 1860

gggaacaagt tcgacatgac ctgcctgccg ctggcctcca ccactcagta cagccgtgca 1920

gtctttgacg ggtgccacta ctactaccta ccctggggta acgtgaaacc tgtggtggtg 1980

ctgtcatcct actgggagga catcagccat agaatcgaga ctcagaacca gctgcttggg 2040

attgctgacc ggctggaagc tggcctggag caggtccacc tggccctgaa ggcgcagtgc 2100

tccacggagg acgtggactc gctggtggct cagctgacgg atgagctcat cgcaggctgc 2160

agccagcctc tgggtgacat ccatgagaca ccctctgcca cccacctgga ccagtacctg 2220

taccagctgc gcacccatca cctgagccaa atcactgagg ctgccctggc cctgaagctc 2280

ggccacagtg agctccctgc agctctggag caggcggagg actggctcct gcgtctgcgt 2340

gccctggcag aggagcccca gaacagcctg ccggacatcg tcatctggat gctgcaggga 2400

gacaagcgtg tggcatacca gcgggtgccc gcccaccaag tcctcttctc ccggcggggt 2460

gccaactact gtggcaagaa ttgtgggaag ctacagacaa tctttctgaa atatccgatg 2520

gagaaggtgc ctggcgcccg gatgccagtg cagatacggg tcaagctgtg gtttgggctc 2580

tctgtggatg agaaggagtt caaccagttt gctgagggga agctgtctgt ctttgctgaa 2640

acctatgaga acgagactaa gttggccctt gttgggaact ggggcacaac gggcctcacc 2700

taccccaagt tttctgacgt cacgggcaag atcaagctac ccaaggacag cttccgcccc 2760

tcggccggct ggacctgggc tggagattgg ttcgtgtgtc cggagaagac tctgctccat 2820

gacatggacg ccggtcacct gagcttcgtg gaagaggtgt ttgagaacca gacccggctt 2880

cccggaggcc agtggatcta catgagtgac aactacaccg atgtgaacgg ggagaaggtg 2940

cttcccaagg atgacattga gtgcccactg ggctggaagt gggaagatga ggaatggtcc 3000

acagacctca accgggctgt cgatgagcaa ggctgggagt atagcatcac catccccccg 3060

gagcggaagc cgaagcactg ggtccctgct gagaagatgt actacacaca ccgacggcgg 3120

cgctgggtgc gcctgcgcag gagggatctc agccaaatgg aagcactgaa aaggcacagg 3180

caggcggagg cggagggcga gggctgggag tacgcctctc tttttggctg gaagttccac 3240

ctcgagtacc gcaagacaga tgccttccgc cgccgccgct ggcgccgtcg catggagcca 3300

ctggagaaga cggggcctgc agctgtgttt gcccttgagg gggccctggg cggatgctga 3360

gggtcttcat cctctatgcc gagaacgtcc acacacccga caccgacatc agcgatgcct 3420

actgctccgc ggtgtttgca ggggtgaaga agagaaccaa agtcatcaag aacagcgtga 3480

accctgtatg gaatgaggga tttgaatggg acctcaaggg catccccctg gaccagggct 3540

ctgagcttca tgtggtggtc aaagaccatg agacgatggg gaggaacagg ttcctggggg 3600

aagccaaggt cccactccga gaggtcctcg ccacccctag tctgtccgcc agcttcaatg 3660

cccccctgct ggacaccaag aagcagccca caggggcctc gctggtcctg caggtgtcct 3720

acacaccgct gcctggagct gtgcccctgt tcccgccccc tactcctctg gagccctccc 3780

cgactctgcc tgacctggat gtagtggcag acacaggagg agaggaagac acagaggacc 3840

agggactcac tggagatgag gcggagccat tcctggatca aagcggaggc ccgggggctc 3900

ccaccacccc aaggaaacta ccttcacgtc ctccgcccca ctaccccggg atcaaaagaa 3960

agcgaagtgc gcctacatct agaaagctgc tgtcagacaa accgcaggat ttccagatca 4020

gggtccaggt gatcgagggg cgccagctgc cgggggtgaa catcaagcct gtggtcaagg 4080

ttaccgctgc agggcagacc aagcggacgc ggatccacaa gggaaacagc ccactcttca 4140

atgagactct tttcttcaac ttgtttgact ctcctgggga gctgtttgat gagcccatct 4200

ttatcacggt ggtagactct cgttctctca ggacagatgc tctcctcggg gagttccgga 4260

tggacgtggg caccatttac agagagcccc ggcacgccta tctcaggaag tggctgctgc 4320

tctcagaccc tgatgacttc tctgctgggg ccagaggcta cctgaaaaca agcctttgtg 4380

tgctggggcc tggggacgaa gcgcctctgg agagaaaaga cccctctgaa gacaaggagg 4440

acattgaaag caacctgctc cggcccacag gcgtagccct gcgaggagcc cacttctgcc 4500

tgaaggtctt ccgggccgag gacttgccgc agatggacga tgccgtgatg gacaacgtga 4560

aacagatctt tggcttcgag agtaacaaga agaacttggt ggaccccttt gtggaggtca 4620

gctttgcggg gaaaatgctg tgcagcaaga tcttggagaa gacggccaac cctcagtgga 4680

accagaacat cacactgcct gccatgtttc cctccatgtg cgaaaaaatg aggattcgta 4740

tcatagactg ggaccgcctg actcacaatg acatcgtggc taccacctac ctgagtatgt 4800

cgaaaatctc tgcccctgga ggagaaatag aagaggagcc tgcaggtgct gtcaagcctt 4860

cgaaagcctc agacttggat gactacctgg gcttcctccc cacttttggg ccctgctaca 4920

tcaacctcta tggcagtccc agagagttca caggcttccc agacccctac acagagctca 4980

acacaggcaa gggggaaggt gtggcttatc gtggccggct tctgctctcc ctggagacca 5040

agctggtgga gcacagtgaa cagaaggtgg aggaccttcc tgcggatgac atcctccggg 5100

tggagaagta ccttaggagg cgcaagtact ccctgtttgc ggccttctac tcagccacca 5160

tgctgcagga tgtggatgat gccatccagt ttgaggtcag catcggaaac tacggaaaca 5220

agttcgacat gacctgcctg ccgctggcct ccaccactca gtacagccgt gcagtctttg 5280

acgggtgcca ctactactac ctaccctggg gtaacgtgaa acctgtggtg gtgctgtcat 5340

cctactggga ggacatcagc catagaatcg agactcagaa ccagctgctt gggattgctg 5400

accggctgga agctggcctg gagcaggtcc acctggccct gaaggcgcag tgctccacgg 5460

aggacgtgga ctcgctggtg gctcagctga cggatgagct catcgcaggc tgcagccagc 5520

ctctgggtga catccatgag acaccctctg ccacccacct ggaccagtac ctgtaccagc 5580

tgcgcaccca tcacctgagc caaatcactg aggctgccct ggccctgaag ctcggccaca 5640

gtgagctccc tgcagctctg gagcaggcgg aggactggct cctgcgtctg cgtgccctgg 5700

cagaggagcc ccagaacagc ctgccggaca tcgtcatctg gatgctgcag ggagacaagc 5760

gtgtggcata ccagcgggtg cccgcccacc aagtcctctt ctcccggcgg ggtgccaact 5820

actgtggcaa gaattgtggg aagctacaga caatctttct gaaatatccg atggagaagg 5880

tgcctggcgc ccggatgcca gtgcagatac gggtcaagct gtggtttggg ctctctgtgg 5940

atgagaagga gttcaaccag tttgctgagg ggaagctgtc tgtctttgct gaaacctatg 6000

agaacgagac taagttggcc cttgttggga actggggcac aacgggcctc acctacccca 6060

agttttctga cgtcacgggc aagatcaagc tacccaagga cagcttccgc ccctcggccg 6120

gctggacctg ggctggagat tggttcgtgt gtccggagaa gactctgctc catgacatgg 6180

acgccggtca cctgagcttc gtggaagagg tgtttgagaa ccagacccgg cttcccggag 6240

gccagtggat ctacatgagt gacaactaca ccgatgtgaa cggggagaag gtgcttccca 6300

aggatgacat tgagtgccca ctgggctgga agtgggaaga tgaggaatgg tccacagacc 6360

tcaaccgggc tgtcgatgag caaggctggg agtatagcat caccatcccc ccggagcgga 6420

agccgaagca ctgggtccct gctgagaaga tgtactacac acaccgacgg cggcgctggg 6480

tgcgcctgcg caggagggat ctcagccaaa tggaagcact gaaaaggcac aggcaggcgg 6540

aggcggaggg cgagggctgg gagtacgcct ctctttttgg ctggaagttc cacctcgagt 6600

accgcaagac agatgccttc cgccgccgcc gctggcgccg tcgcatggag ccactggaga 6660

agacggggcc tgcagctgtg tttgcccttg agggggccct gggcggcgtg atggatgaca 6720

agagtgaaga ttccatgtcc gtctccacct tgagcttcgg tgtgaacaga cccacgattt 6780

cctgcatatt cgactatggg aaccgctacc atctacgctg ctacatgtac caggcccggg 6840

acctggctgc gatggacaag gactcttttt ctgatcccta tgccatcgtc tccttcctgc 6900

accagagcca gaagacggtg gtggtgaaga acacccttaa ccccacctgg gaccagacgc 6960

tcatcttcta cgagatcgag atctttggcg agccggccac agttgctgag caaccgccca 7020

gcattgtggt ggagctgtac gaccatgaca cttatggtgc agacgagttt atgggtcgct 7080

gcatctgtca accgagtctg gaacggatgc cacggctggc ctggttccca ctgacgaggg 7140

gcagccagcc gtcgggggag ctgctggcct cttttgagct catccagaga gagaagccgg 7200

ccatccacca tattcctggt tttgaggtgc aggagacatc aaggatcctg gatgagtctg 7260

aggacacaga cctgccctac ccaccacccc agagggaggc caacatctac atggttcctc 7320

agaacatcaa gccagcgctc cagcgtaccg ccatcgagat cctggcatgg ggcctgcgga 7380

acatgaagag ttaccagctg gccaacatct cctcccccag cctcgtggta gagtgtgggg 7440

gccagacggt gcagtcctgt gtcatcagga acctccggaa gaaccccaac tttgacatct 7500

gcaccctctt catggaagtg atgctgccca gggaggagct ctactgcccc cccatcaccg 7560

tcaaggtcat cgataaccgc cagtttggcc gccggcctgt ggtgggccag tgtaccatcc 7620

gctccctgga gagcttcctg tgtgacccct actcggcgga gagtccatcc ccacagggtg 7680

gcccagacga tgtgagccta ctcagtcctg gggaagacgt gctcatcgac attgatgaca 7740

aggagcccct catccccatc caggaggaag agttcatcga ttggtggagc aaattctttg 7800

cctccatagg ggagagggaa aagtgcggct cctacctgga gaaggatttt gacaccctga 7860

aggtctatga cacacagctg gagaatgtgg aggcctttga gggcctgtct gacttttgta 7920

acaccttcaa gctgtaccgg ggcaagacgc aggaggagac agaagatcca tctgtgattg 7980

gtgaatttaa gggcctcttc aaaatttatc ccctcccaga agacccagcc atccccatgc 8040

ccccaagaca gttccaccag ctggccgccc agggacccca ggagtgcttg gtccgtatct 8100

acattgtccg agcatttggc ctgcagccca aggaccccaa tggaaagtgt gatccttaca 8160

tcaagatctc catagggaag aaatcagtga gtgaccagga taactacatc ccctgcacgc 8220

tggagcccgt atttggaaag atgttcgagc tgacctgcac tctgcctctg gagaaggacc 8280

taaagatcac tctctatgac tatgacctcc tctccaagga cgaaaagatc ggtgagacgg 8340

tcgtcgacct ggagaacagg ctgctgtcca agtttggggc tcgctgtgga ctcccacaga 8400

cctactgtgt ctctggaccg aaccagtggc gggaccagct ccgcccctcc cagctcctcc 8460

acctcttctg ccagcagcat agagtcaagg cacctgtgta ccggacagac cgtgtaatgt 8520

ttcaggataa agaatattcc attgaagaga tagaggctgg caggatccca aacccacacc 8580

tgggcccagt ggaggagcgt ctggctctgc atgtgcttca gcagcagggc ctggtcccgg 8640

agcacgtgga gtcacggccc ctctacagcc ccctgcagcc agacatcgag caggggaagc 8700

tgcagatgtg ggtcgaccta tttccgaagg ccctggggcg gcctggacct cccttcaaca 8760

tcaccccacg gagagccaga aggtttttcc tgcgttgtat tatctggaat accagagatg 8820

tgatcctgga tgacctgagc ctcacggggg agaagatgag cgacatttat gtgaaaggtt 8880

ggatgattgg ctttgaagaa cacaagcaaa agacagacgt gcattatcgt tccctgggag 8940

gtgaaggcaa cttcaactgg aggttcattt tccccttcga ctacctgcca gctgagcaag 9000

tctgtaccat tgccaagaag gatgccttct ggaggctgga caagactgag agcaaaatcc 9060

cagcacgagt ggtgttccag atctgggaca atgacaagtt ctcctttgat gattttctgg 9120

gctccctgca gctcgatctc aaccgcatgc ccaagccagc caagacagcc aagaagtgct 9180

ccttggacca gctggatgat gctttccacc cagaatggtt tgtgtccctt tttgagcaga 9240

aaacagtgaa gggctggtgg ccctgtgtag cagaagaggg tgagaagaaa atactggcgg 9300

gcaagctgga aatgaccttg gagattgtag cagagagtga gcatgaggag cggcctgctg 9360

gccagggccg ggatgagccc aacatgaacc ctaagcttga ggacccaagg cgccccgaca 9420

cctccttcct gtggtttacc tccccataca agaccatgaa gttcatcctg tggcggcgtt 9480

tccggtgggc catcatcctc ttcatcatcc tcttcatcct gctgctgttc ctggccatct 9540

tcatctacgc cttcccgaac tatgctgcca tgaagctggt gaagcccttc agctga 9596

<210> 32
<211> 6306
<212> DNA
<213> Artificial

<220>
<223> Nucleotide sequence for dysferlin 11

<400> 32
atgctgaggg tcttcatcct ctatgccgag aacgtccaca cacccgacac cgacatcagc 60

gatgcctact gctccgcggt gtttgcaggg gtgaagaaga gaaccaaagt catcaagaac 120

agcgtgaacc ctgtatggaa tgagggattt gaatgggacc tcaagggcat ccccctggac 180

cagggctctg agcttcatgt ggtggtcaaa gaccatgaga cgatggggag gaacaggttc 240

ctgggggaag ccaaggtccc actccgagag gtcctcgcca cccctagtct gtccgccagc 300

ttcaatgccc ccctgctgga caccaagaag cagcccacag gggcctcgct ggtcctgcag 360

gtgtcctaca caccgctgcc tggagctgtg cccctgttcc cgccccctac tcctctggag 420

ccctccccga ctctgcctga cctggatgta gtggcagaca caggaggaga ggaagacaca 480

gaggaccagg gactcactgg agatgaggcg gagccattcc tggatcaaag cggaggcccg 540

ggggctccca ccaccccaag gaaactacct tcacgtcctc cgccccacta ccccgggatc 600

aaaagaaagc gaagtgcgcc tacatctaga aagctgctgt cagacaaacc gcaggatttc 660

cagatcaggg tccaggtgat cgaggggcgc cagctgccgg gggtgaacat caagcctgtg 720

gtcaaggtta ccgctgcagg gcagaccaag cggacgcgga tccacaaggg aaacagccca 780

ctcttcaatg agactctttt cttcaacttg tttgactctc ctggggagct gtttgatgag 840

cccatcttta tcacggtggt agactctcgt tctctcagga cagatgctct cctcggggag 900

ttccggatgg acgtgggcac catttacaga gagccccggc acgcctatct caggaagtgg 960

ctgctgctct cagaccctga tgacttctct gctggggcca gaggctacct gaaaacaagc 1020

ctttgtgtgc tggggcctgg ggacgaagcg cctctggaga gaaaagaccc ctctgaagac 1080

aaggaggaca ttgaaagcaa cctgctccgg cccacaggcg tagccctgcg aggagcccac 1140

ttctgcctga aggtcttccg ggccgaggac ttgccgcaga tggacgatgc cgtgatggac 1200

aacgtgaaac agatctttgg cttcgagagt aacaagaaga acttggtgga cccctttgtg 1260

gaggtcagct ttgcggggaa aatgctgtgc agcaagatct tggagaagac ggccaaccct 1320

cagtggaacc agaacatcac actgcctgcc atgtttccct ccatgtgcga aaaaatgagg 1380

attcgtatca tagactggga ccgcctgact cacaatgaca tcgtggctac cacctacctg 1440

agtatgtcga aaatctctgc ccctggagga gaaatagaag aggagcctgc aggtgctgtc 1500

aagccttcga aagcctcaga cttggatgac tacctgggct tcctccccac ttttgggccc 1560

tgctacatca acctctatgg cagtcccaga gagttcacag gcttcccaga cccctacaca 1620

gagctcaaca caggcaaggg ggaaggtgtg gcttatcgtg gccggcttct gctctccctg 1680

gagaccaagc tggtggagca cagtgaacag aaggtggagg accttcctgc ggatgacatc 1740

ctccgggtgg agaagtacct taggaggcgc aagtactccc tgtttgcggc cttctactca 1800

gccaccatgc tgcaggatgt ggatgatgcc atccagtttg aggtcagcat cgggaactac 1860

gggaacaagt tcgacatgac ctgcctgccg ctggcctcca ccactcagta cagccgtgca 1920

gtctttgacg ggtgccacta ctactaccta ccctggggta acgtgaaacc tgtggtggtg 1980

ctgtcatcct actgggagga catcagccat agaatcgaga ctcagaacca gctgcttggg 2040

attgctgacc ggctggaagc tggcctggag caggtccacc tggccctgaa ggcgcagtgc 2100

tccacggagg acgtggactc gctggtggct cagctgacgg atgagctcat cgcaggctgc 2160

agccagcctc tgggtgacat ccatgagaca ccctctgcca cccacctgga ccagtacctg 2220

taccagctgc gcacccatca cctgagccaa atcactgagg ctgccctggc cctgaagctc 2280

ggccacagtg agctccctgc agctctggag caggcggagg actggctcct gcgtctgcgt 2340

gccctggcag aggagcccca gaacagcctg ccggacatcg tcatctggat gctgcaggga 2400

gacaagcgtg tggcatacca gcgggtgccc gcccaccaag tcctcttctc ccggcggggt 2460

gccaactact gtggcaagaa ttgtgggaag ctacagacaa tctttctgaa atatccgatg 2520

gagaaggtgc ctggcgcccg gatgccagtg cagatacggg tcaagctgtg gtttgggctc 2580

tctgtggatg agaaggagtt caaccagttt gctgagggga agctgtctgt ctttgctgaa 2640

acctatgaga acgagactaa gttggccctt gttgggaact ggggcacaac gggcctcacc 2700

taccccaagt tttctgacgt cacgggcaag atcaagctac ccaaggacag cttccgcccc 2760

tcggccggct ggacctgggc tggagattgg ttcgtgtgtc cggagaagac tctgctccat 2820

gacatggacg ccggtcacct gagcttcgtg gaagaggtgt ttgagaacca gacccggctt 2880

cccggaggcc agtggatcta catgagtgac aactacaccg atgtgaacgg ggagaaggtg 2940

cttcccaagg atgacattga gtgcccactg ggctggaagt gggaagatga ggaatggtcc 3000

acagacctca accgggctgt cgatgagcaa ggctgggagt atagcatcac catccccccg 3060

gagcggaagc cgaagcactg ggtccctgct gagaagatgt actacacaca ccgacggcgg 3120

cgctgggtgc gcctgcgcag gagggatctc agccaaatgg aagcactgaa aaggcacagg 3180

caggcggagg cggagggcga gggctgggag tacgcctctc tttttggctg gaagttccac 3240

ctcgagtacc gcaagacaga tgccttccgc cgccgccgct ggcgccgtcg catggagcca 3300

ctggagaaga cggggcctgc agctgtgttt gcccttgagg gggccctggg cggcgtgatg 3360

gatgacaaga gtgaagattc catgtccgtc tccaccttga gcttcggtgt gaacagaccc 3420

acgatttcct gcatattcga ctatgggaac cgctaccatc tacgctgcta catgtaccag 3480

gcccgggacc tggctgcgat ggacaaggac tctttttctg atccctatgc catcgtctcc 3540

ttcctgcacc agagccagaa gacggtggtg gtgaagaaca cccttaaccc cacctgggac 3600

cagacgctca tcttctacga gatcgagatc tttggcgagc cggccacagt tgctgagcaa 3660

ccgcccagca ttgtggtgga gctgtacgac catgacactt atggtgcaga cgagtttatg 3720

ggtcgctgca tctgtcaacc gagtctggaa cggatgccac ggctggcctg gttcccactg 3780

acgaggggca gccagccgtc gggggagctg ctggcctctt ttgagctcat ccagagagag 3840

aagccggcca tccaccatat tcctggtttt gaggtgcagg agacatcaag gatcctggat 3900

gagtctgagg acacagacct gccctaccca ccaccccaga gggaggccaa catctacatg 3960

gttcctcaga acatcaagcc agcgctccag cgtaccgcca tcgagatcct ggcatggggc 4020

ctgcggaaca tgaagagtta ccagctggcc aacatctcct cccccagcct cgtggtagag 4080

tgtgggggcc agacggtgca gtcctgtgtc atcaggaacc tccggaagaa ccccaacttt 4140

gacatctgca ccctcttcat ggaagtgatg ctgcccaggg aggagctcta ctgccccccc 4200

atcaccgtca aggtcatcga taaccgccag tttggccgcc ggcctgtggt gggccagtgt 4260

accatccgct ccctggagag cttcctgtgt gacccctact cggcggagag tccatcccca 4320

cagggtggcc cagacgatgt gagcctactc agtcctgggg aagacgtgct catcgacatt 4380

gatgacaagg agcccctcat ccccatccag cttgcagacg gtctgtcgag cttggccccc 4440

actaacacgg cttctcctcc atccagtcct catgaggaag agttcatcga ttggtggagc 4500

aaattctttg cctccatagg ggagagggaa aagtgcggct cctacctgga gaaggatttt 4560

gacaccctga aggtctatga cacacagctg gagaatgtgg aggcctttga gggcctgtct 4620

gacttttgta acaccttcaa gctgtaccgg ggcaagacgc aggaggagac agaagatcca 4680

tctgtgattg gtgaatttaa gggcctcttc aaaatttatc ccctcccaga agacccagcc 4740

atccccatgc ccccaagaca gttccaccag ctggccgccc agggacccca ggagtgcttg 4800

gtccgtatct acattgtccg agcatttggc ctgcagccca aggaccccaa tggaaagtgt 4860

gatccttaca tcaagatctc catagggaag aaatcagtga gtgaccagga taactacatc 4920

ccctgcacgc tggagcccgt atttggaaag atgttcgagc tgacctgcac tctgcctctg 4980

gagaaggacc taaagatcac tctctatgac tatgacctcc tctccaagga cgaaaagatc 5040

ggtgagacgg tcgtcgacct ggagaacagg ctgctgtcca agtttggggc tcgctgtgga 5100

ctcccacaga cctactgtgt ctctggaccg aaccagtggc gggaccagct ccgcccctcc 5160

cagctcctcc acctcttctg ccagcagcat agagtcaagg cacctgtgta ccggacagac 5220

cgtgtaatgt ttcaggataa agaatattcc attgaagaga tagaggctgg caggatccca 5280

aacccacacc tgggcccagt ggaggagcgt ctggctctgc atgtgcttca gcagcagggc 5340

ctggtcccgg agcacgtgga gtcacggccc ctctacagcc ccctgcagcc agacatcgag 5400

caggggaagc tgcagatgtg ggtcgaccta tttccgaagg ccctggggcg gcctggacct 5460

cccttcaaca tcaccccacg gagagccaga aggtttttcc tgcgttgtat tatctggaat 5520

accagagatg tgatcctgga tgacctgagc ctcacggggg agaagatgag cgacatttat 5580

gtgaaaggtt ggatgattgg ctttgaagaa cacaagcaaa agacagacgt gcattatcgt 5640

tccctgggag gtgaaggcaa cttcaactgg aggttcattt tccccttcga ctacctgcca 5700

gctgagcaag tctgtaccat tgccaagaag gatgccttct ggaggctgga caagactgag 5760

agcaaaatcc cagcacgagt ggtgttccag atctgggaca atgacaagtt ctcctttgat 5820

gattttctgg gctccctgca gctcgatctc aaccgcatgc ccaagccagc caagacagcc 5880

aagaagtgct ccttggacca gctggatgat gctttccacc cagaatggtt tgtgtccctt 5940

tttgagcaga aaacagtgaa gggctggtgg ccctgtgtag cagaagaggg tgagaagaaa 6000

atactggcgg gcaagctgga aatgaccttg gagattgtag cagagagtga gcatgaggag 6060

cggcctgctg gccagggccg ggatgagccc aacatgaacc ctaagcttga ggacccaagg 6120

cgccccgaca cctccttcct gtggtttacc tccccataca agaccatgaa gttcatcctg 6180

tggcggcgtt tccggtgggc catcatcctc ttcatcatcc tcttcatcct gctgctgttc 6240

ctggccatct tcatctacgc cttcccgaac tatgctgcca tgaagctggt gaagcccttc 6300

agctga 6306